(19)

Europäisches
Patentamt
European
Patent Office
Office européen
des brevets

(11)  **EP 3 965 115 A1**

(12)  **EUROPEAN PATENT APPLICATION**

(43) Date of publication:
     **09.03.2022  Bulletin 2022/10**

(51) International Patent Classification (IPC):
     **G16H 20/40** *(2018.01)*

(21) Application number: **21194504.3**

(52) Cooperative Patent Classification (CPC):
     **G16H 20/40**

(22) Date of filing: **02.09.2021**

(84) Designated Contracting States:
     **AL AT BE BG CH CY CZ DE DK EE ES FI FR GB
     GR HR HU IE IS IT LI LT LU LV MC MK MT NL NO
     PL PT RO RS SE SI SK SM TR**
     Designated Extension States:
     **BA ME**
     Designated Validation States:
     **KH MA MD TN**

(30) Priority: **02.09.2020  US 202063073530 P
                30.08.2021  US 202117461651**

(71) Applicant: **Biosense Webster (Israel) Ltd
     Yokneam, 2066717 (IL)**

(72) Inventors:
     • **AUERBACH, Shmuel**
       **Yokneam (IL)**
     • **ELIYAHU, Shiran**
       **Yokneam (IL)**
     • **KAUFMAN, Ana**
       **Yokneam (IL)**

(74) Representative: **Carpmaels & Ransford LLP
     One Southampton Row
     London WC1B 5HA (GB)**

(54)  **AUTOMATICALLY IDENTIFYING SCAR AREAS WITHIN ORGANIC TISSUE USING MULTIPLE
      IMAGING MODALITIES**

(57)  A system and method for aiding a physician in locating the origin of an arrythmia for patients with non-sustained tachycardia are disclosed. The system and method includes receiving data at a machine, from at least one device, the data including information relating to a desired location for performing an ablation, generating, by the machine, an optimal location for performing the ablation based upon the data and inputs, and providing an optimal location for performing the ablation output by the model. The output may include a certainty score for the origins of the arrythmia. The output includes an option to obtain new origins during the ablation procedure.

**EP 3 965 115 A1**

**Description**

CROSS REFERENCE TO RELATED APPLICATION(S)

This application claims the benefit of U.S. Provisional Application No. 63/073,530, filed September 2, 2020, the contents of which are incorporated herein by reference.

FIELD OF INVENTION

The present invention is related to artificial intelligence and machine learning associated with predicting the origins and required steps of a cardiac ablation procedure, intervention for patient specific electrophysiology, and ablation strategy related to cardiac arrhythmia treatment determination.

BACKGROUND

The origins of arrythmia are difficult to predict when the medical condition is intermittent (i.e. not stable). A common method to identify the sources when a patient is exhibiting normal heart rhythm is to pace the heart into an abnormal rhythm which is similar to the medical condition. This approach is challenging in a large subset of the patients due to the cardiac burden it generates.

BRIEF DESCRIPTION OF THE DRAWINGS

A more detailed understanding may be had from the following description, given by way of example in conjunction with the accompanying drawings, wherein like reference numerals in the figures indicate like elements, and wherein:

FIG. 1 is a block diagram of an example system for remotely monitoring and communicating patient biometrics;
FIG. 2 is a system diagram of an example of a computing environment in communication with network;
FIG. 3 is a block diagram of an example device in which one or more features of the disclosure can be implemented;
FIG. 4 illustrates a graphical depiction of an artificial intelligence system incorporating the example device of FIG. 3;
FIG. 5 illustrates a method performed in the artificial intelligence system of FIG. 4;
FIG. 6 illustrates an example of the probabilities of a naive Bayes calculation;
FIG. 7 illustrates an exemplary decision tree;
FIG. 8 illustrates an exemplary random forest classifier;
FIG. 9 illustrates an exemplary logistic regression;
FIG. 10 illustrates an exemplary support vector machine;
FIG. 11 illustrated an exemplary linear regression model;
FIG. 12 illustrates an exemplary K-means clustering;
FIG. 13 illustrates an exemplary ensemble learning algorithm;
FIG. 14 illustrates an exemplary neural network;
FIG. 15 illustrates a hardware based neural network;
FIG. 16A illustrates the bipolar signal amplitude (Bi) variance in the various sectors of the heart;
FIG. 16B illustrates the Shortex Complex Interval (SCI) variance in the various sectors of the heart;
FIGs. 16C illustrates an epicardial voltage map of a heart experiencing left ventricular non-compaction cardiomyopathy;
FIG. 16D illustrates a potential duration map (PDM) of a heart experiencing left ventricular non-compaction cardiomyopathy;
FIG. 17 is a diagram of an exemplary system 1720 in which one or more features of the disclosure subject matter can be implemented;
FIG. 18 illustrates a heart;
FIG. 19 illustrates a graphical depiction of the artificial intelligence system of FIG. 4 providing further detail related to predicting the steps of a cardiac ablation procedure;
FIG. 20 illustrates a system using a neural network in accordance with the embodiments described herein;
FIG. 21 illustrates a neural network in accordance with the embodiments described herein; and
FIG. 22 illustrates an illustration of the use of the systems described herein.

DETAILED DESCRIPTION

A system and method for aiding a physician in locating the origin of an arrythmia for patients with non-sustained tachycardia are disclosed. The system and method includes receiving data at a machine, from at least one device, the data including information relating to a desired location for performing an ablation, generating, by the machine, an optimal location for performing the ablation based upon the data and inputs, and providing an optimal location for performing the ablation output by the model. The output may include a certainty score for the origins of the arrythmia. The output includes an option to obtain new origins during the ablation procedure.

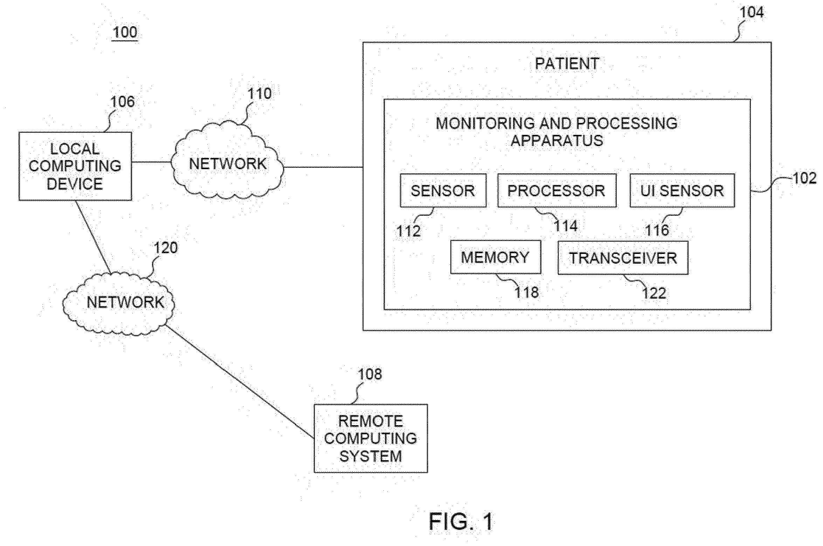

FIG. 1 is a block diagram of an example system 100 for remotely monitoring and communicating patient biometrics (i.e., patient data). In the example illustrated in FIG. 1, the system 100 includes a patient biometric monitoring and processing apparatus 102 associated with a patient 104, a local computing device 106, a remote computing system 108, a first network 110 and a second network 120.

According to an embodiment, a monitoring and processing apparatus 102 may be an apparatus that is internal to the patient's body (e.g., subcutaneously implantable). The monitoring and processing apparatus 102 may be inserted into a patient via any applicable manner including orally injecting, surgical insertion via a vein or artery, an endoscopic procedure, or a laparoscopic procedure.

According to an embodiment, a monitoring and processing apparatus 102 may be an apparatus that is external to the patient. For example, as described in more detail below, the monitoring and processing apparatus 102 may include an attachable patch (e.g., that attaches to a patient's skin). The monitoring and processing apparatus 102 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an embodiment, a monitoring and processing apparatus 102 may include both components that are internal to the patient and components that are external to the patient.

A single monitoring and processing apparatus 102 is shown in FIG. 1. Example systems may, however, may include a plurality of patient biometric monitoring and processing apparatuses. A patient biometric monitoring and processing apparatus may be in communication with one or more other patient biometric monitoring and processing apparatuses. Additionally, or alternatively, a patient biometric monitoring and processing apparatus may be in communication with the network 110.

One or more monitoring and processing apparatuses 102 may acquire patient biometric data (e.g., electrical signals, blood pressure, temperature, blood glucose level or other biometric data) and receive at least a portion of the patient biometric data representing the acquired patient biometrics and additional formation associated with acquired patient biometrics from one or more other monitoring and processing apparatuses 102. The additional information may be, for example, diagnosis information and/or additional information obtained from an additional device such as a wearable device. Each monitoring and processing apparatus 102 may process data, including its own acquired patient biometrics as well as data received from one or more other monitoring and processing apparatuses 102.

In FIG. 1, network 110 is an example of a short-range network (e.g., local area network (LAN), or personal area network (PAN)). Information may be sent, via short-range network 110, between monitoring a processing apparatus 102 and local computing device 106 using any one of various short-range wireless communication protocols, such as Bluetooth, Wi-Fi, Zigbee, Z-Wave, near field communications (NFC), ultraband, Zigbee, or infrared (IR).

Network 120 may be a wired network, a wireless network or include one or more wired and wireless networks. For example, a network 120 may be a long-range network (e.g., wide area network (WAN), the internet, or a cellular network,). Information may be sent, via network 120 using any one of various long-range wireless communication protocols (e.g., TCP/IP, HTTP, 3G, 4G/LTE, or 5G/New Radio).

The patient monitoring and processing apparatus 102 may include a patient biometric sensor 112, a processor 114, a user input (UI) sensor 116, a memory 118, and a transmitter-receiver (i.e., transceiver) 122. The patient monitoring and processing apparatus 102 may continually or periodically monitor, store, process and communicate, via network 110, any number of various patient biometrics. Examples of patient biometrics include electrical signals (e.g., ECG signals and brain biometrics), blood pressure data, blood glucose data and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

Patient biometric sensor 112 may include, for example, one or more sensors configured to sense a type of biometric patient biometrics. For example, patient biometric sensor 112 may include an electrode configured to acquire electrical signals (e.g., heart signals, brain signals or other bioelectrical signals), a temperature sensor, a blood pressure sensor, a blood glucose sensor, a blood oxygen sensor, a pH sensor, an accelerometer and a microphone.

As described in more detail below, patient biometric monitoring and processing apparatus 102 may be an ECG monitor for monitoring ECG signals of a heart. The patient biometric sensor 112 of the ECG monitor may include one or more electrodes for acquiring ECG signals. The ECG signals may be used for treatment of various cardiovascular diseases.

In another example, the patient biometric monitoring and processing apparatus 102 may be a continuous glucose monitor (CGM) for continuously monitoring blood glucose levels of a patient on a continual basis for treatment of various diseases, such as type I and type II diabetes. The CGM may include a subcutaneously disposed electrode, which may monitor blood glucose levels from interstitial fluid of the patient. The CGM may be, for example, a component of a closed-loop system in which the blood glucose data is sent to an insulin pump for calculated delivery of insulin without user intervention.

Transceiver 122 may include a separate transmitter and receiver. Alternatively, transceiver 122 may include a transmitter and receiver integrated into a single device.

Processor 114 may be configured to store patient data, such as patient biometric data in memory 118 acquired by patient biometric sensor 112, and communicate the patient data, across network 110, via a transmitter of transceiver 122. Data from one or more other monitoring and processing apparatus 102 may also be received by a receiver of transceiver 122, as described in more detail below.

According to an embodiment, the monitoring and processing apparatus 102 includes UI sensor 116 which may be, for example, a piezoelectric sensor or a capacitive sensor configured to receive a user input, such as a tapping or touching. For example, UI sensor 116 may be controlled to implement a capacitive coupling, in response to tapping or touching a surface of the monitoring and processing apparatus 102 by the patient 104. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface such that the tapping or touching of the surface activates the monitoring device.

As described in more detail below, the processor 114 may be configured to respond selectively to different tapping patterns of the capacitive sensor (e.g., a single tap or a double tap), which may be the UI sensor 116, such that different tasks of the patch (e.g., acquisition, storing, or transmission of data) may be activated based on the detected pattern. In some embodiments, audible feedback may be given to the user from processing apparatus 102 when a gesture is detected.

The local computing device 106 of system 100 is in communication with the patient biometric monitoring and processing apparatus 102 and may be configured to act as a gateway to the remote computing system 108 through the second network 120. The local computing device 106 may be, for example, a, smart phone, smartwatch, tablet or other portable smart device configured to communicate with other devices via network 120. Alternatively, the local computing device 106 may be a stationary or standalone device, such as a stationary base station including, for example, modem and/or router capability, a desktop or laptop computer using an executable program to communicate information between the processing apparatus 102 and the remote computing system 108 via the PC's radio module, or a USB dongle. Patient biometrics may be communicated between the local computing device 106 and the patient biometric monitoring and processing apparatus 102 using a short-range wireless technology standard (e.g., Bluetooth, Wi-Fi, ZigBee, Z-wave and other short-range wireless standards) via the short-range wireless network 110, such as a local area network (LAN) (e.g., a personal area network (PAN)). In some embodiments, the local computing device 106 may also be configured to display the acquired patient electrical signals and information associated with the acquired patient electrical signals, as described in more detail below.

In some embodiments, remote computing system 108 may be configured to receive at least one of the monitored patient biometrics and information associated with the monitored patient via network 120, which is a long-range network. For example, if the local computing device 106 is a mobile phone, network 120 may be a wireless cellular network, and information may be communicated between the local computing device 106 and the remote computing system 108 via a wireless technology standard, such as any of the wireless technologies mentioned above. As described in more detail below, the remote computing system 108 may be configured to provide (e.g., visually display and/or aurally provide) the at least one of the patient biometrics and the associated information to a healthcare professional (e.g., a physician).

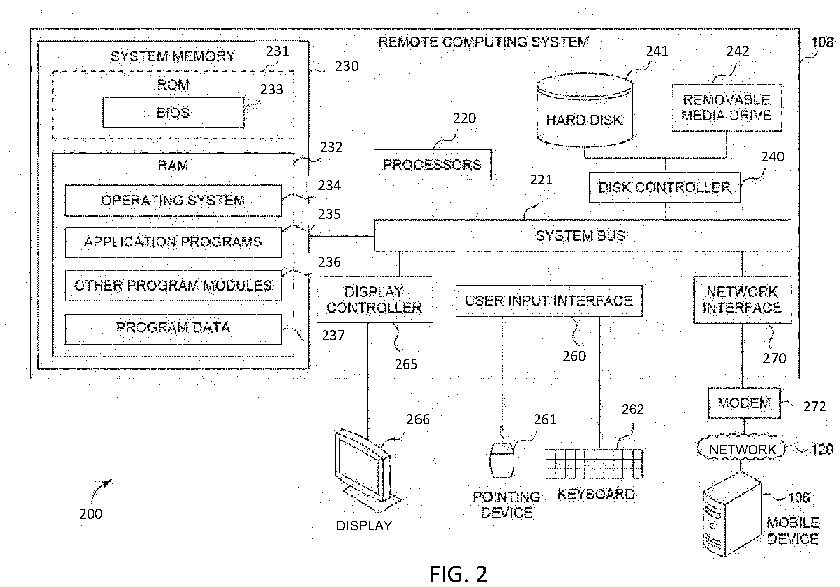

FIG. 2 is a system diagram of an example of a computing environment 200 in communication with network 120. In some instances, the computing environment 200 is incorporated in a public cloud computing platform (such as Amazon Web Services or Microsoft Azure), a hybrid cloud computing platform (such as HP Enterprise OneSphere) or a private cloud computing platform.

As shown in FIG. 2, computing environment 200 includes remote computing system 108 (hereinafter computer system), which is one example of a computing system upon which embodiments described herein may be implemented.

The remote computing system 108 may, via processors 220, which may include one or more processors, perform various functions. The functions may include analyzing monitored patient biometrics and the associated information and, according to physician-determined or algorithm driven thresholds and parameters, providing (e.g., via display 266) alerts, additional information or instructions. As described in more detail below, the remote computing system 108 may be used to provide (e.g., via display 266) healthcare personnel (e.g., a physician) with a dashboard of patient information, such that such information may enable healthcare personnel to identify and prioritize patients having more critical needs than others.

As shown in FIG. 2, the computer system 210 may include a communication mechanism such as a bus 221 or other communication mechanism for communicating information within the computer system 210. The computer system 210 further includes one or more processors 220 coupled with the bus 221 for processing the information. The processors 220 may include one or more CPUs, GPUs, or any other processor known in the art.

The computer system 210 also includes a system memory 230 coupled to the bus 221 for storing information and instructions to be executed by processors 220. The system memory 230 may include computer readable storage media in the form of volatile and/or nonvolatile memory, such as read only system memory (ROM) 231 and/or random-access memory (RAM) 232. The system memory RAM 232 may include other dynamic storage device(s) (e.g., dynamic RAM, static RAM, and synchronous DRAM). The system memory ROM 231 may include other static storage device(s) (e.g., programmable ROM, erasable PROM, and electrically erasable PROM). In addition, the system memory 230 may be used for storing temporary variables or other intermediate information during the execution of instructions by the processors 220. A basic input/output system 233 (BIOS) may contain routines to transfer information between elements within computer system 210, such as during start-up, that may be stored in system memory ROM 231. RAM 232 may comprise data and/or program modules that are immediately accessible to and/or presently being operated on by the processors 220. System memory 230 may additionally include, for example, operating system 234, application programs 235, other program modules 236 and program data 237.

The illustrated computer system 210 also includes a disk controller 240 coupled to the bus 221 to control one or more storage devices for storing information and instructions, such as a magnetic hard disk 241 and a removable media drive 242 (e.g., floppy disk drive, compact disc drive, tape drive, and/or solid-state drive). The storage devices may be added to the computer system 210 using an appropriate device interface (e.g., a small computer system interface (SCSI), integrated device electronics (IDE), Universal Serial Bus (USB), or FireWire).

The computer system 210 may also include a display controller 265 coupled to the bus 221 to control a monitor or display 266, such as a cathode ray tube (CRT) or liquid crystal display (LCD), for displaying information to a computer user. The illustrated computer system 210 includes a user input interface 260 and one or more input devices, such as a keyboard 262 and a pointing device 261, for interacting with a computer user and providing information to the processor 220. The pointing device 261, for example, may be a mouse, a trackball, or a pointing stick for communicating direction information and command selections to the processor 220 and for controlling cursor movement on the display 266. The display 266 may provide a touch screen interface that may allow input to supplement or replace the communication of direction information and command selections by the pointing device 261 and/or keyboard 262.

The computer system 210 may perform a portion or each of the functions and methods described herein in response to the processors 220 executing one or more sequences of one or more instructions contained in a memory, such as the system memory 230. Such instructions may be read into the system memory 230 from another computer readable medium, such as a hard disk 241 or a removable media drive 242. The hard disk 241 may contain one or more data stores and data files used by embodiments described herein. Data store contents and data files may be encrypted to improve security. The processors 220 may also be employed in a multiprocessing arrangement to execute the one or more sequences of instructions contained in system memory 230. In alternative embodiments, hard-wired circuitry may be used in place of or in combination with software instructions. Thus, embodiments are not limited to any specific combination of hardware circuitry and software.

As stated above, the computer system 210 may include at least one computer readable medium or memory for holding instructions programmed according to embodiments described herein and for containing data structures, tables, records, or other data described herein. The term computer readable medium as used herein refers to any non-transitory, tangible medium that participates in providing instructions to the processor 220 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 241 or removable media drive 242. Non-limiting examples of volatile media include dynamic memory, such as system memory 230. Non-limiting examples of transmission media include coaxial cables, copper wire, and fiber optics, including the wires that make up the bus 221. Transmission media may also take the form of acoustic or light waves, such as those generated during radio wave and infrared data communications.

The computing environment 200 may further include the computer system 210 operating in a networked environment using logical connections to local computing device 106 and one or more other devices, such as a personal computer (laptop or desktop), mobile devices (e.g., patient mobile devices), a server, a router, a network PC, a peer device or other common network node, and typically includes many or all of the elements described above relative to computer system 210. When used in a networking environment, computer system 210 may include modem 272 for establishing communications over a network 120, such as the Internet. Modem 272 may be connected to system bus 221 via network interface 270, or via another appropriate mechanism.

Network 120, as shown in FIGs. 1 and 2, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 610 and other computers (e.g., local computing device 106).

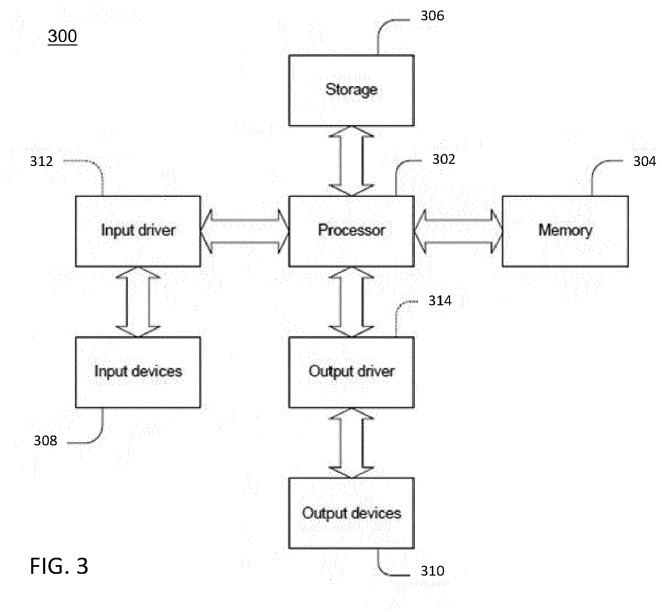

FIG. 3 is a block diagram of an example device 300 in which one or more features of the disclosure can be implemented. The device 300 may be local computing device 106, for example. The device 300 can include, for example, a computer, a gaming device, a handheld device, a set-top box, a television, a mobile phone, or a tablet computer. The device 300 includes a processor 302, a memory 304, a storage device 306, one or more input devices 308, and one or more output devices 310. The device 300 can also optionally include an input driver 312 and an output driver 314. It is understood that the device 300 can include additional components not shown in FIG. 3 including an artificial intelligence accelerator.

In various alternatives, the processor 302 includes a central processing unit (CPU), a graphics processing unit (GPU), a CPU and GPU located on the same die, or one or more processor cores, wherein each processor core can be a CPU or a GPU. In various alternatives, the memory 304 is located on the same die as the processor 302, or is located separately from the processor 302. The memory 304 includes a volatile or non-volatile memory, for example, random access memory (RAM), dynamic RAM, or a cache.

The storage device 306 includes a fixed or removable storage means, for example, a hard disk drive, a solid-state drive, an optical disk, or a flash drive. The input devices 308 include, without limitation, a keyboard, a keypad, a touch screen, a touch pad, a detector, a microphone, an accelerometer, a gyroscope, a biometric scanner, or a network connection (e.g., a wireless local area network card for transmission and/or reception of wireless IEEE 802 signals). The output devices 310 include, without limitation, a display, a speaker, a printer, a haptic feedback device, one or more lights, an antenna, or a network connection (e.g., a wireless local area network card for transmission and/or reception of wireless IEEE 802 signals).

The input driver 312 communicates with the processor 302 and the input devices 308, and permits the processor 302 to receive input from the input devices 308. The output driver 314 communicates with the processor 302 and the output devices 310, and permits the processor 302 to send output to the output devices 310. It is noted that the input driver 312 and the output driver 314 are optional components, and that the device 300 will operate in the same manner if the input driver 312 and the output driver 314 are not present. The output driver 316 includes an accelerated processing device ("APD") 316 which is coupled to a display device 318. The APD accepts compute commands and graphics rendering commands from processor 302, processes those compute and graphics rendering commands, and provides pixel output to display device 318 for display. As described in further detail below, the APD 316 includes one or more parallel processing units to perform computations in accordance with a single-instruction-multiple-data ("SIMD") paradigm. Thus, although various functionality is described herein as being performed by or in conjunction with the APD 316, in various alternatives, the functionality described as being performed by the APD 316 is additionally or alternatively performed by other computing devices having similar capabilities that are not driven by a host processor (e.g., processor 302) and provides graphical output to a display device 318. For example, it is contemplated that any processing system that performs processing tasks in accordance with a SIMD paradigm may perform the functionality described herein. Alternatively, it is contemplated that computing systems that do not perform processing tasks in accordance with a SIMD paradigm performs the functionality described herein.

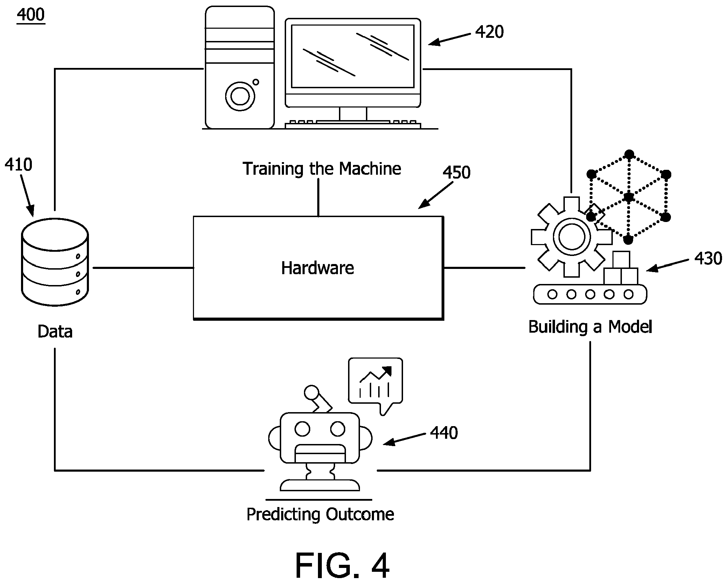

FIG. 4 illustrates a graphical depiction of an artificial intelligence system 200 incorporating the example device of FIG. 3. System 400 includes data 410, a machine 420, a model 430, a plurality of outcomes 440 and underlying hardware 450. System 400 operates by using the data 410 to train the machine 420 while building a model 430 to enable a plurality of outcomes 440 to be predicted. The system 400 may operate with respect to hardware 450. In such a configuration, the data 410 may be related to hardware 450 and may originate with apparatus 102, for example. For example, the data 410 may be on-going data, or output data associated with hardware 450. The machine 420 may operate as the controller or data collection associated with the hardware 450, or be associated therewith. The model 430 may be configured to model the operation of hardware 450 and model the data 410 collected from hardware 450 in order to predict the outcome achieved by hardware 450. Using the outcome 440 that is predicted, hardware 450 may be configured to provide a certain desired outcome 440 from hardware 450.

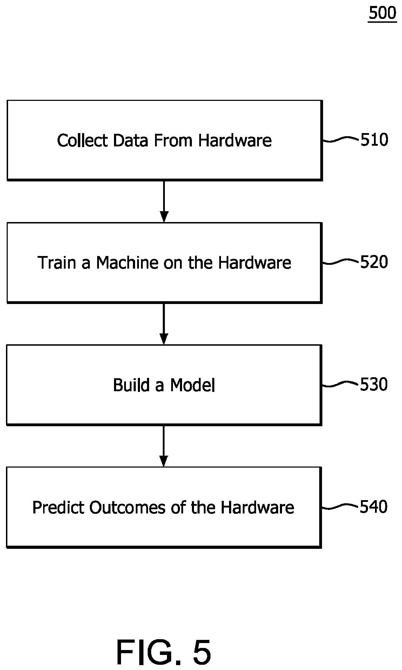

FIG. 5 illustrates a method 500 performed in the artificial intelligence system of FIG. 4. Method 500 includes collecting data from the hardware at step 510. This data may include currently collected, historical or other data from the hardware. For example, this data may include measurements during a surgical procedure and may be associated with the outcome of the procedure. For example, the temperature of a heart may be collected and correlated with the outcome of a heart procedure.

At step 520, method 500 includes training a machine on the hardware. The training may include an analysis and correlation of the data collected in step 510. For example, in the case of the heart, the data of temperature and outcome may be trained to determine if a correlation or link exists between the temperature of the heart during the procedure and the outcome.

At step 530, method 500 includes building a model on the data associated with the hardware. Building a model may include physical hardware or software modeling, algorithmic modeling and the like, as will be described below. This modeling may seek to represent the data that has been collected and trained.

At step 540, method 500 includes predicting the outcomes of the model associated with the hardware. This prediction of the outcome may be based on the trained model. For example, in the case of the heart, if the temperature during the procedure between 97.7 - 100.2 produces a positive result from the procedure, the outcome can be predicted in a given procedure based on the temperature of the heart during the procedure. While this model is rudimentary, it is provided for exemplary purposes and to increase understanding of the present invention.

The present system and method operate to train the machine, build the model and predict outcomes using algorithms. These algorithms may be used to solve the trained model and predict outcomes associated with the hardware. These algorithms may be divided generally into classification, regression and clustering algorithms.

For example, a classification algorithm is used in the situation where the dependent variable, which is the variable being predicted, is divided into classes and predicting a class, the dependent variable, for a given input. Thus, a classification algorithm is used to predict an outcome, from a set number of fixed, predefined outcomes. A classification algorithm may include naive Bayes algorithms, decision trees, random forest classifiers, logistic regressions, support vector machines and k nearest neighbors.

Generally, a naive Bayes algorithm follows the Bayes theorem, and follows a probabilistic approach. As would be understood, other probabilistic-based algorithms may also be used, and generally operate using similar probabilistic principles to those described below for the exemplary naive Bayes algorithm.

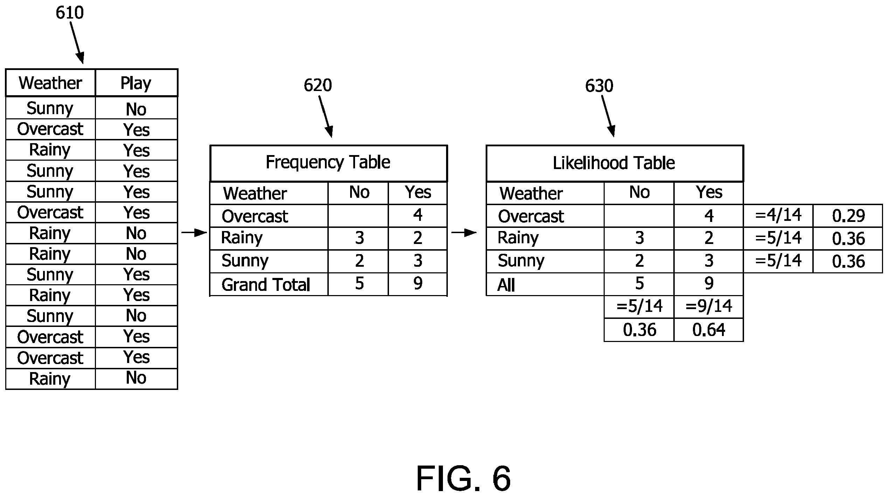

FIG. 6 illustrates an example of the probabilities of a naive Bayes calculation. The probability approach of Bayes theorem essentially means, that instead of jumping straight into the data, the algorithm has a set of prior probabilities for each of the classes for the target. After the data is entered, the naive Bayes algorithm may update the prior probabilities to form a posterior probability. This is given by the formula:

$$posterior = \frac{prior \; x \; likelihood}{evidence}$$

This naive Bayes algorithm, and Bayes algorithms generally, may be useful when needing to predict whether your input belongs to a given list of n classes or not. The probabilistic approach may be used because the probabilities for all the n classes will be quite low.

For example, as illustrated in FIG. 6, a person playing golf, which depends on factors including the weather outside shown in a first data set 610. The first data set 610 illustrates the weather in a first column and an outcome of playing associated with that weather in a second column. In the frequency table 620 the frequencies with which certain events occur are generated. In frequency table 620, the frequency of a person playing or not playing golf in each of the weather conditions is determined. From there, a likelihood table is compiled to generate initial probabilities. For example, the probability of the weather being overcast is 0.29 while the general probability of playing is 0.64.

The posterior probabilities may be generated from the likelihood table 630. These posterior probabilities may be configured to answer questions about weather conditions and whether golf is played in those weather conditions. For example, the probability of it being sunny outside and golf being played may be set forth by the Bayesian formula:

$$P(Yes \mid Sunny) = P(Sunny \mid Yes) * P(Yes) / P(Sunny)$$

According to likelihood table 630:

$$P(Sunny \mid Yes) = 3/9 = 0.33,$$

$$P(Sunny) = 5/14 = 0.36,$$

$$P(Yes) = 9/14 = 0.64.$$

Therefore, the P(Yes | Sunny) = .33*.64/.36 or approximately 0.60 (60%).

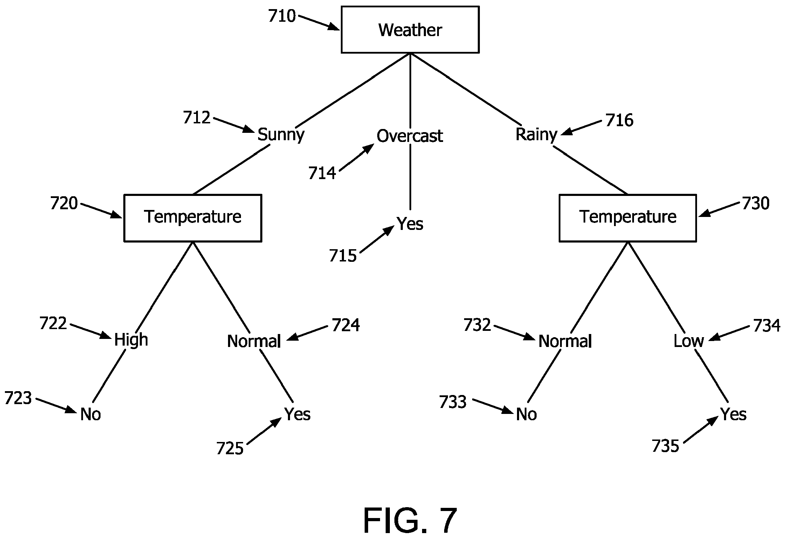

Generally, a decision tree is a flowchart-like tree structure where each external node denotes a test on an attribute and each branch represents the outcome of that test. The leaf nodes contain the actual predicted labels. The decision tree begins from the root of the tree with attribute values being compared until a leaf node is reached. A decision tree can be used as a classifier when handling high dimensional data and when little time has been spent behind data preparation. Decision trees may take the form of a simple decision tree, a linear decision tree, an algebraic decision tree, a deterministic decision tree, a randomized decision tree, a nondeterministic decision tree, and a quantum decision tree. An exemplary decision tree is provided below in FIG. 7.

FIG. 7 illustrates a decision tree, along the same structure as the Bayes example above, in deciding whether to play golf. In the decision tree, the first node 710 examines the weather providing sunny 712, overcast 714, and rain 716 as the choices to progress down the decision tree. If the weather is sunny, the leg of the tree is followed to a second node 720 examining the temperature. The temperature at node 720 may be high 722 or normal 724, in this example. If the temperature at node 720 is high 722, then the predicted outcome of "No" 723 golf occurs. If the temperature at node 720 is normal 724, then the predicted outcome of "Yes" 725 golf occurs.

Further, from the first node 710, an outcome overcast 714, "Yes" 715 golf occurs.

From the first node weather 710, an outcome of rain 716 results in the third node 730 (again) examining temperature. If the temperature at third node 730 is normal 732, then "Yes" 733 golf is played. If the temperature at third node 730 is low 734, then "No" 735 golf is played.

From this decision tree, a golfer plays golf if the weather is overcast 715, in normal temperature sunny weather 725, and in normal temperature rainy weather 733, while the golfer does not play if there are sunny high temperatures 723 or low rainy temperatures 735.

A random forest classifier is a committee of decision trees, where each decision tree has been fed a subset of the attributes of data and predicts on the basis of that subset. The mode of the actual predicted values of the decision trees are considered to provide an ultimate random forest answer. The random forest classifier, generally, alleviates overfitting, which is present in a standalone decision tree, leading to a much more robust and accurate classifier.

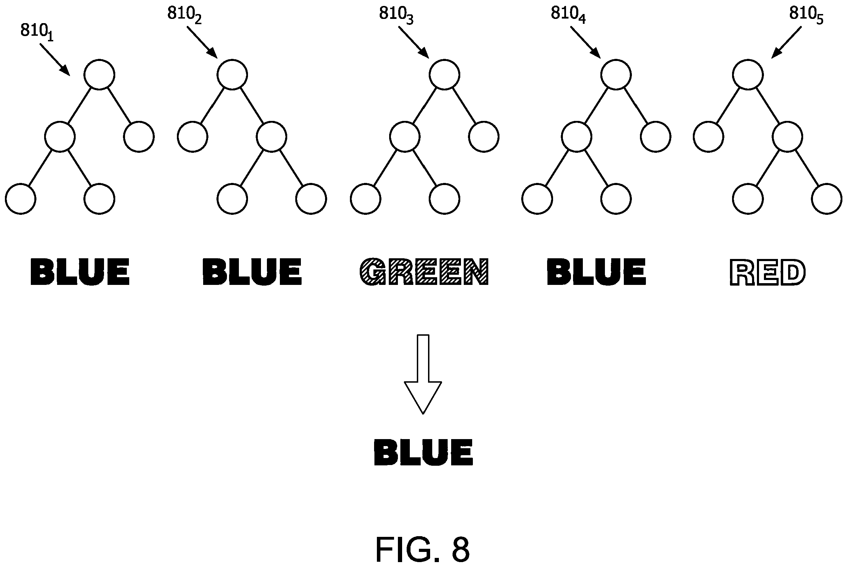

FIG. 8 illustrates an exemplary random forest classifier for classifying the color of a garment. As illustrated in FIG. 8, the random forest classifier includes five decision trees $810_1$, $810_2$, $810_3$, $810_4$, and $810_5$ (collectively or generally referred to as decision trees 810). Each of the trees is designed to classify the color of the garment. A discussion of each of the trees and decisions made is not provided, as each individual tree generally operates as the decision tree of FIG. 7. In the illustration, three ($810_1$, $810_2$, $810_4$) of the five trees determines that the garment is blue, while one determines the garment is green ($810_3$) and the remaining tree determines the garment is red ($810_5$). The random forest takes these actual predicted values of the five trees and calculates the mode of the actual predicted values to provide random forest answer that the garment is blue.

Logistic Regression is another algorithm for binary classification tasks. Logistic regression is based on the logistic function, also called the sigmoid function. This S-shaped curve can take any real-valued number and map it between 0 and 1 asymptotically approaching those limits. The logistic model may be used to model the probability of a certain class or event existing such as pass/fail, win/lose, alive/dead or healthy/sick. This can be extended to model several classes of events such as determining whether an image contains a cat, dog, lion, etc. Each object being detected in the image would be assigned a probability between 0 and 1 with the sum of the probabilities adding to one.

In the logistic model, the log-odds (the logarithm of the odds) for the value labeled "1" is a linear combination of one or more independent variables ("predictors"); the independent variables can each be a binary variable (two classes, coded by an indicator variable) or a continuous variable (any real value). The corresponding probability of the value labeled "1" can vary between 0 (certainly the value "0") and 1 (certainly the value "1"), hence the labeling; the function that converts log-odds to probability is the logistic function, hence the name. The unit of measurement for the log-odds scale is called a logit, from logistic unit, hence the alternative names. Analogous models with a different sigmoid function instead of the logistic function can also be used, such as the probit model; the defining characteristic of the logistic model is that increasing one of the independent variables multiplicatively scales the odds of the given outcome at a constant rate, with each independent variable having its own parameter; for a binary dependent variable this generalizes the odds ratio.

In a binary logistic regression model, the dependent variable has two levels (categorical). Outputs with more than two values are modeled by multinomial logistic regression and, if the multiple categories are ordered, by ordinal logistic regression (for example the proportional odds ordinal logistic model). The logistic regression model itself simply models probability of output in terms of input and does not perform statistical classification (it is not a classifier), though it can be used to make a classifier, for instance by choosing a cutoff value and classifying inputs with probability greater than the cutoff as one class, below the cutoff as the other; this is a common way to make a binary classifier.

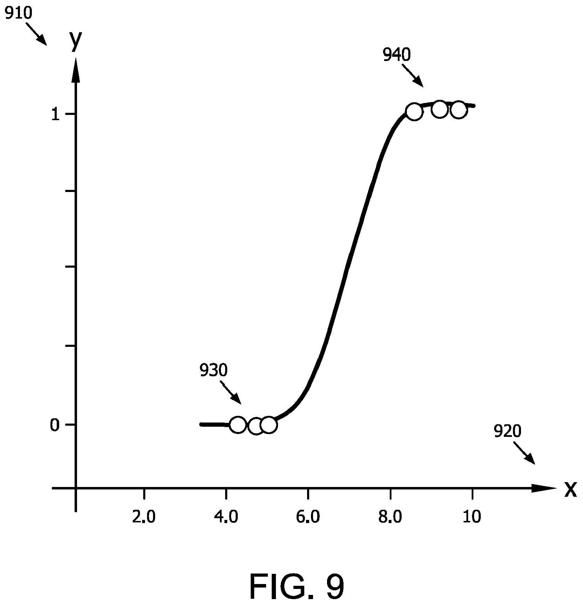

FIG. 9 illustrates an exemplary logistic regression. This exemplary logistic regression enables the prediction of an outcome based on a set of variables. For example, based on a person's grade point average, and outcome of being accepted to a school may be predicted. The past history of grade point averages and the relationship with acceptance enables the prediction to occur. The logistic regression of FIG. 9 enables the analysis of the grade point average variable 920 to predict the outcome 910 defined by 0 to 1. At the low end 930 of the S-shaped curve, the grade point average 920 predicts an outcome 910 of not being accepted. While at the high end 940 of the S-shaped curve, the grade point average 920 predicts an outcome 910 of being accepted. Logistic regression may be used to predict house values, customer lifetime value in the insurance sector, etc.

A support vector machine (SVM) may be used to sort the data with the margins between two classes as far apart as possible. This is called maximum margin separation. The SVM may account for the support vectors while plotting the hyperplane, unlike linear regression which uses the entire dataset for that purpose.

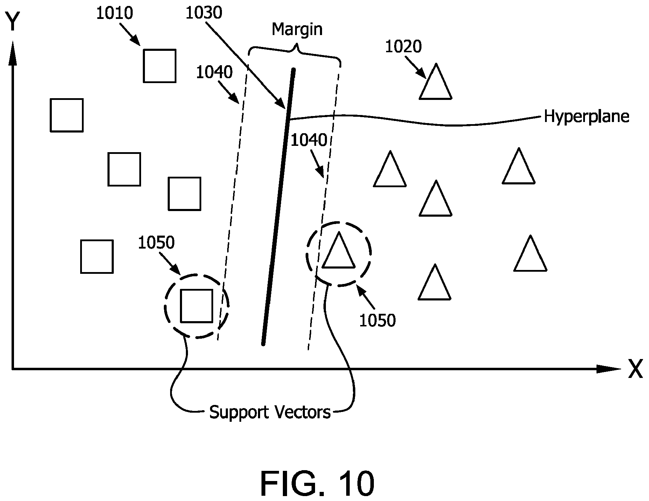

FIG. 10 illustrates an exemplary support vector machine. In the exemplary SVM 1000, data may be classified into two different classes represented as squares 1010 and triangles 1020. SVM 1000 operates by drawing a random hyperplane 1030. This hyperplane 1030 is monitored by comparing the distance (illustrated with lines 1040) between the hyperplane 1030 and the closest data points 1050 from each class. The closest data points 1050 to the hyperplane 1030 are known as support vectors. The hyperplane 1030 is drawn based on these support vectors 1050 and an optimum hyperplane has a maximum distance from each of the support vectors 1050. The distance between the hyperplane 1030 and the support vectors 1050 is known as the margin.

SVM 1000 may be used to classify data by using a hyperplane 1030, such that the distance between the hyperplane 1030 and the support vectors 1050 is maximum. Such an SVM 1000 may be used to predict heart disease, for example.

K Nearest Neighbors (KNN) refers to a set of algorithms that generally do not make assumptions on the underlying data distribution, and perform a reasonably short training phase. Generally, KNN uses many data points separated into several classes to predict the classification of a new sample point. Operationally, KNN specifies an integer N with a new sample. The N entries in the model of the system closest to the new sample are selected. The most common classification of these entries is determined and that classification is assigned to the new sample. KNN generally requires the storage space to increase as the training set increases. This also means that the estimation time increases in proportion to the number of training points.

In regression algorithms, the output is a continuous quantity so regression algorithms may be used in cases where the target variable is a continuous variable. Linear regression is a general example of regression algorithms. Linear regression may be used to gauge genuine qualities (cost of houses, number of calls, all out deals and so forth) in view of the consistent variable(s). A connection between the variables and the outcome is created by fitting the best line (hence linear regression). This best fit line is known as regression line and spoken to by a direct condition $Y= a\ {}^*X + b$. Linear regression is best used in approaches involving a low number of dimensions

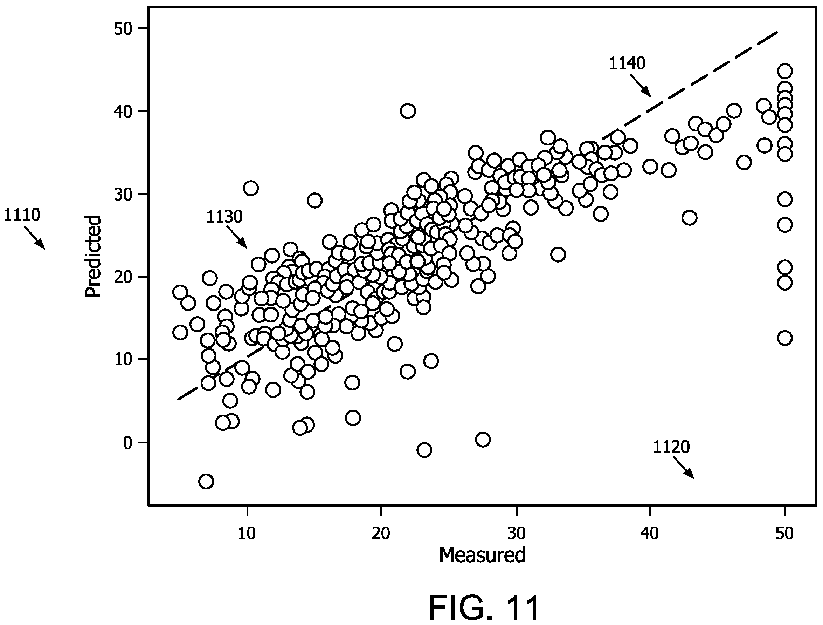

FIG. 11 illustrates an exemplary linear regression model. In this model, a predicted variable 1110 is modeled against a measured variable 1120. A cluster of instances of the predicted variable 1110 and measured variable 1120 are plotted as data points 1130. Data points 1130 are then fit with the best fit line 1140. Then the best fit line 1140 is used in subsequent predicted, given a measured variable 1120, the line 1140 is used to predict the predicted variable 1110 for that instance. Linear regression may be used to model and predict in a financial portfolio, salary forecasting, real estate and in traffic in arriving at estimated time of arrival.

Clustering algorithms may also be used to model and train on a data set. In clustering, the input is assigned into two or more clusters based on feature similarity. Clustering algorithms generally learn the patterns and useful insights from data without any guidance. For example, clustering viewers into similar groups based on their interests, age, geography, etc. may be performed using unsupervised learning algorithms like K-means clustering.

K-means clustering generally is regarded as a simple unsupervised learning approach. In K-means clustering similar data points may be gathered together and bound in the form of a cluster. One method for binding the data points together is by calculating the centroid of the group of data points. In determining effective clusters, in K-means clustering the distance between each point from the centroid of the cluster is evaluated. Depending on the distance between the data point and the centroid, the data is assigned to the closest cluster. The goal of clustering is to determine the intrinsic grouping in a set of unlabeled data. The 'K' in K-means stands for the number of clusters formed. The number of clusters (basically the number of classes in which new instances of data may be classified) may be determined by the user. This determination may be performed using feedback and viewing the size of the clusters during training, for example.

K-means is used majorly in cases where the data set has points which are distinct and well separated, otherwise, if the clusters are not separated the modeling may render the clusters inaccurate. Also, K-means may be avoided in cases where the data set contains a high number of outliers or the data set is non-linear.

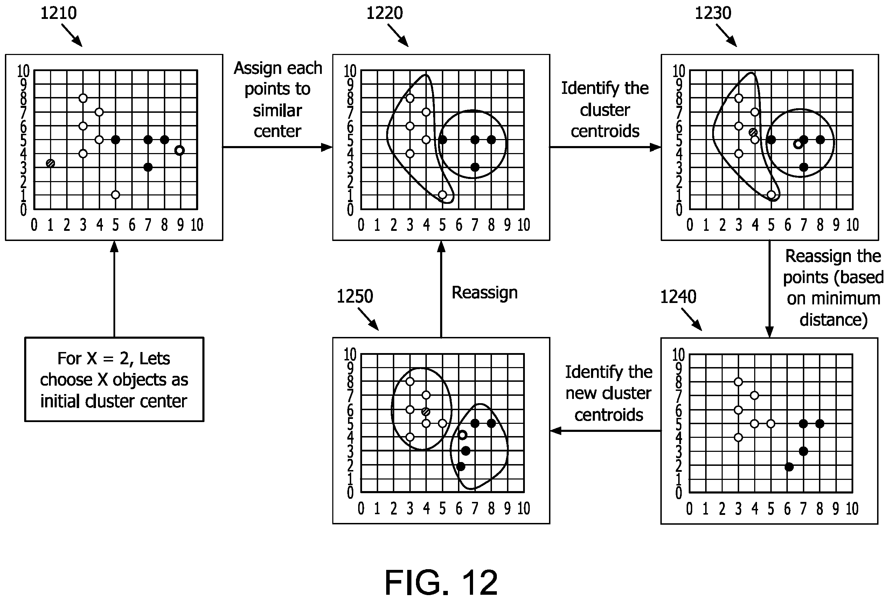

FIG. 12 illustrates a K-means clustering. In K-means clustering, the data points are plotted and the K value is assigned. For example, for K=2 in FIG. 12, the data points are plotted as shown in depiction 1210. The points are then assigned to similar centers at step 1220. The cluster centroids are identified as shown in 1230. Once centroids are identified, the points are reassigned to the cluster to provide the minimum distance between the data point to the respective cluster centroid as illustrated in 1240. Then a new centroid of the cluster may be determined as illustrated in depiction 1250. As the data pints are reassigned to a cluster, new cluster centroids formed, an iteration, or series of iterations, may occur to enable the clusters to be minimized in size and the centroid of the optimal centroid determined. Then as new data points are measured, the new data points may be compared with the centroid and cluster to identify with that cluster.

Ensemble learning algorithms may be used. These algorithms use multiple learning algorithms to obtain better predictive performance than could be obtained from any of the constituent learning algorithms alone. Ensemble learning algorithms perform the task of searching through a hypothesis space to find a suitable hypothesis that will make good predictions with a particular problem. Even if the hypothesis space contains hypotheses that are very well-suited for a particular problem, it may be very difficult to find a good hypothesis. Ensemble algorithms combine multiple hypotheses to form a better hypothesis. The term ensemble is usually reserved for methods that generate multiple hypotheses using the same base learner. The broader term of multiple classifier systems also covers hybridization of hypotheses that are not induced by the same base learner.

Evaluating the prediction of an ensemble typically requires more computation than evaluating the prediction of a single model, so ensembles may be thought of as a way to compensate for poor learning algorithms by performing a lot of extra computation. Fast algorithms such as decision trees are commonly used in ensemble methods, for example, random forests, although slower algorithms can benefit from ensemble techniques as well.

An ensemble is itself a supervised learning algorithm, because it can be trained and then used to make predictions. The trained ensemble, therefore, represents a single hypothesis. This hypothesis, however, is not necessarily contained within the hypothesis space of the models from which it is built. Thus, ensembles can be shown to have more flexibility in the functions they can represent. This flexibility can, in theory, enable them to over-fit the training data more than a single model would, but in practice, some ensemble techniques (especially bagging) tend to reduce problems related to over-fitting of the training data.

Empirically, ensemble algorithms tend to yield better results when there is a significant diversity among the models. Many ensemble methods, therefore, seek to promote diversity among the models they combine. Although non-intuitive, more random algorithms (like random decision trees) can be used to produce a stronger ensemble than very deliberate algorithms (like entropy-reducing decision trees). Using a variety of strong learning algorithms, however, has been shown to be more effective than using techniques that attempt to dumb-down the models in order to promote diversity.

The number of component classifiers of an ensemble has a great impact on the accuracy of prediction. *A priori* determining of ensemble size and the volume and velocity of big data streams make this even more crucial for online ensemble classifiers. A theoretical framework suggests that there are an ideal number of component classifiers for an ensemble such that having more or less than this number of classifiers would deteriorate the accuracy. The theoretical framework shows that using the same number of independent component classifiers as class labels gives the highest accuracy.

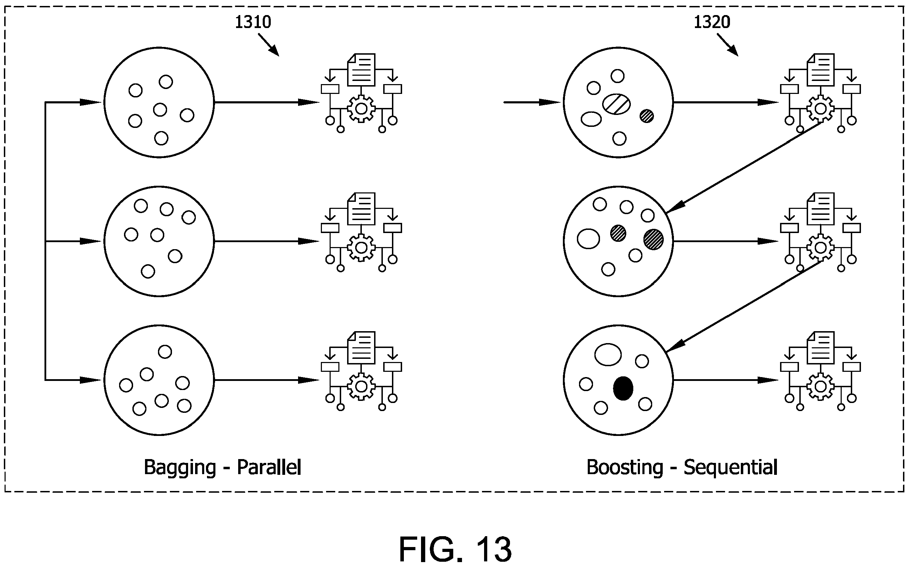

Some common types of ensembles include Bayes optimal classifier, bootstrap aggregating (bagging), boosting, Bayesian model averaging, Bayesian model combination, bucket of models and stacking. FIG. 13 illustrates an exemplary ensemble learning algorithm where bagging is being performed in parallel 1310 and boosting is being performed sequentially 1320.

A neural network is a network or circuit of neurons, or in a modern sense, an artificial neural network, composed of artificial neurons or nodes. The connections of the biological neuron are modeled as weights. A positive weight reflects an excitatory connection, while negative values mean inhibitory connections. Inputs are modified by a weight and summed using a linear combination. An activation function may control the amplitude of the output. For example, an acceptable range of output is usually between 0 and 1, or it could be -1 and 1.

These artificial networks may be used for predictive modeling, adaptive control and applications and can be trained via a dataset. Self-learning resulting from experience can occur within networks, which can derive conclusions from a complex and seemingly unrelated set of information.

For completeness, a biological neural network is composed of a group or groups of chemically connected or functionally associated neurons. A single neuron may be connected to many other neurons and the total number of neurons and connections in a network may be extensive. Connections, called synapses, are usually formed from axons to dendrites, though dendrodendritic synapses and other connections are possible. Apart from the electrical signaling, there are other forms of signaling that arise from neurotransmitter diffusion.

Artificial intelligence, cognitive modeling, and neural networks are information processing paradigms inspired by the way biological neural systems process data. Artificial intelligence and cognitive modeling try to simulate some properties of biological neural networks. In the artificial intelligence field, artificial neural networks have been applied successfully to speech recognition, image analysis and adaptive control, in order to construct software agents (in computer and video games) or autonomous robots.

A neural network (NN), in the case of artificial neurons called artificial neural network (ANN) or simulated neural network (SNN), is an interconnected group of natural or artificial neurons that uses a mathematical or computational model for information processing based on a connectionistic approach to computation. In most cases an ANN is an adaptive system that changes its structure based on external or internal information that flows through the network. In more practical terms neural networks are non-linear statistical data modeling or decision-making tools. They can be used to model complex relationships between inputs and outputs or to find patterns in data.

An artificial neural network involves a network of simple processing elements (artificial neurons) which can exhibit complex global behavior, determined by the connections between the processing elements and element parameters.

One classical type of artificial neural network is the recurrent Hopfield network. The utility of artificial neural network models lies in the fact that they can be used to infer a function from observations and also to use it. Unsupervised neural networks can also be used to learn representations of the input that capture the salient characteristics of the input distribution, and more recently, deep learning algorithms, which can implicitly learn the distribution function of the observed data. Learning in neural networks is particularly useful in applications where the complexity of the data or task makes the design of such functions by hand impractical.

Neural networks can be used in different fields. The tasks to which artificial neural networks are applied tend to fall within the following broad categories: function approximation, or regression analysis, including time series prediction and modeling; classification, including pattern and sequence recognition, novelty detection and sequential decision making, data processing, including filtering, clustering, blind signal separation and compression.

Application areas of ANNs include nonlinear system identification and control (vehicle control, process control), game-playing and decision making (backgammon, chess, racing), pattern recognition (radar systems, face identification, object recognition), sequence recognition (gesture, speech, handwritten text recognition), medical diagnosis, financial applications, data mining (or knowledge discovery in databases, "KDD"), visualization and e-mail spam filtering. For example, it is possible to create a semantic profile of user's interests emerging from pictures trained for object recognition.

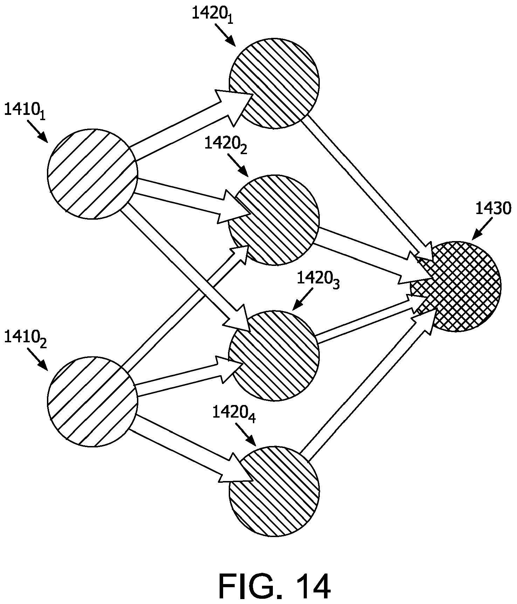

FIG. 14 illustrates an exemplary neural network. In the neural network there is an input layer represented by a plurality of inputs, such as $1410_1$ and $1410_2$. The inputs $1410_1$, $1410_2$ are provided to a hidden layer depicted as including nodes $1420_1$, $1420_2$, $1420_3$, $1420_4$. These nodes $1420_1$, $1420_2$, $1420_3$, $1420_4$ are combined to produce an output 1430 in an output layer. The neural network performs simple processing via the hidden layer of simple processing elements, nodes $1420_1$, $1420_2$, $1420_3$, $1420_4$, which can exhibit complex global behavior, determined by the connections between the processing elements and element parameters.

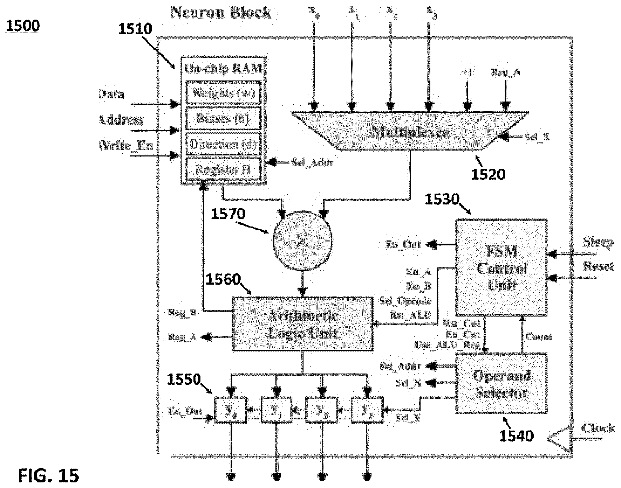

The neural network of FIG. 14 may be implemented in hardware. As depicted in FIG. 15 a hardware based neural network is depicted.

Cardiac arrhythmias, and atrial fibrillation in particular, persist as common and dangerous medical ailments, especially in the aging population. In patients with normal sinus rhythm, the heart, which is comprised of atrial, ventricular, and excitatory conduction tissue, is electrically excited to beat in a synchronous, patterned fashion. In patients with cardiac arrythmias, abnormal regions of cardiac tissue do not follow the synchronous beating cycle associated with normally conductive tissue as in patients with normal sinus rhythm. Instead, the abnormal regions of cardiac tissue aberrantly conduct to adjacent tissue, thereby disrupting the cardiac cycle into an asynchronous cardiac rhythm. Such abnormal conduction has been previously known to occur at various regions of the heart, for example, in the region of the sino-atrial (SA) node, along the conduction pathways of the atrioventricular (AV) node and the Bundle of His, or in the cardiac muscle tissue forming the walls of the ventricular and atrial cardiac chambers.

Cardiac arrhythmias, including atrial arrhythmias, may be of a multiwavelet reentrant type, characterized by multiple asynchronous loops of electrical impulses that are scattered about the atrial chamber and are often self-propagating. Alternatively, or in addition to the multiwavelet reentrant type, cardiac arrhythmias may also have a focal origin, such as when an isolated region of tissue in an atrium fires autonomously in a rapid, repetitive fashion. Ventricular tachycardia (V-tach or VT) is a tachycardia, or fast heart rhythm that originates in one of the ventricles of the heart. This is a potentially life-threatening arrhythmia because it may lead to ventricular fibrillation and sudden death.

One type of arrhythmia, atrial fibrillation, occurs when the normal electrical impulses generated by the sinoatrial node are overwhelmed by disorganized electrical impulses that originate in the atria and pulmonary veins causing irregular impulses to be conducted to the ventricles. An irregular heartbeat results and may last from minutes to weeks, or even years. Atrial fibrillation (AF) is often a chronic condition that leads to a small increase in the risk of death often due to strokes. Risk increases with age. Approximately 8% of people over 80 having some amount of AF. Atrial fibrillation is often asymptomatic and is not in itself generally life-threatening, but it may result in palpitations, weakness, fainting, chest pain and congestive heart failure. Stroke risk increases during AF because blood may pool and form clots in the poorly contracting atria and the left atrial appendage. The first line of treatment for AF is medication that either slow the heart rate or revert the heart rhythm back to normal. Additionally, persons with AF are often given anticoagulants to protect them from the risk of stroke. The use of such anticoagulants comes with its own risk of internal bleeding. In some patients, medication is not sufficient and their AF is deemed to be drug-refractory, i.e., untreatable with standard pharmacological interventions. Synchronized electrical cardioversion may also be used to convert AF to a normal heart rhythm. Alternatively, AF patients are treated by catheter ablation.

A catheter ablation-based treatment may include mapping the electrical properties of heart tissue, especially the endocardium and the heart volume, and selectively ablating cardiac tissue by application of energy. Cardiac mapping, for example, creating a map of electrical potentials (a voltage map) of the wave propagation along the heart tissue or a map of arrival times (a local time activation (LAT) map) to various tissue located points, may be used for detecting local heart tissue dysfunction Ablations, such as those based on cardiac mapping, can cease or modify the propagation of unwanted electrical signals from one portion of the heart to another.

The ablation process damages the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure-mapping followed by ablation-electrical activity at points within the heart is typically sensed and measured by advancing a catheter containing one or more electrical sensors (or electrodes) into the heart, and acquiring data at a multiplicity of points. These data are then utilized to select the endocardial target areas at which ablation is to be performed.

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems in order to reconstruct the anatomy of the heart chamber of interest.

For example, cardiologists rely upon software such as the Complex Fractionated Atrial Electrograms (CFAE) module of the CARTO®3 3D mapping system, produced by Biosense Webster, Inc. (Diamond Bar, Calif.), to analyze intracardiac EGM signals and determine the ablation points for treatment of a broad range of cardiac conditions, including atypical atrial flutter and ventricular tachycardia.

The 3D maps can provide multiple pieces of information regarding the electrophysiological properties of the tissue that represent the anatomical and functional substrate of these challenging arrhythmias.

Cardiomyopathies with different etiologies (ischemic, dilated cardiomyopathy (DCM), hypertrophic cardiomyopathy (HCM), arrhythmogenic right ventricular dysplasia (ARVD), left ventricular non-compaction (LVNC), etc.) have an identifiable substrate, featured by areas of unhealthy tissue surrounded by areas of normally functioning cardiomyocytes.

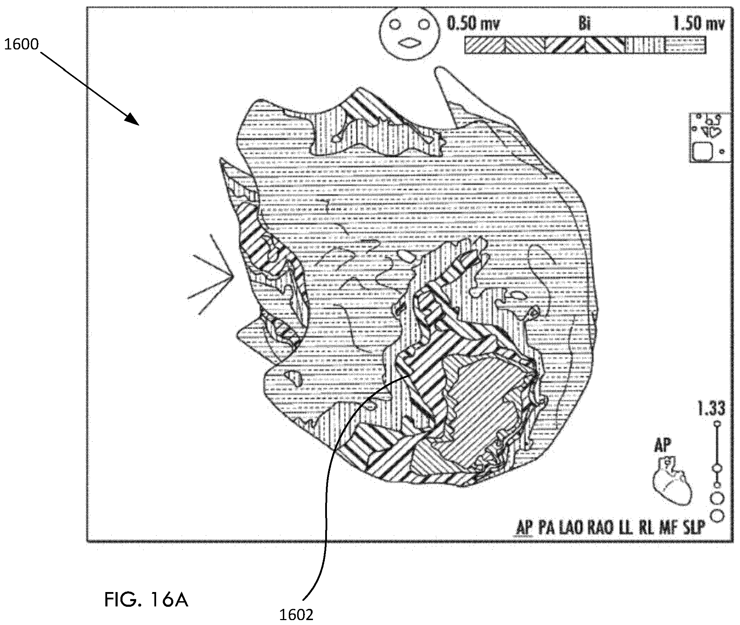

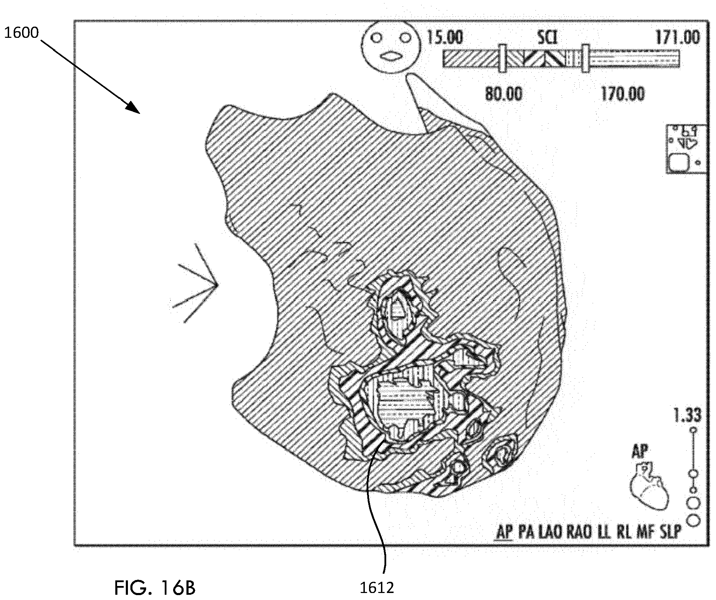

FIGS. 16A through 16D show examples of cardiomyopathies with different etiologies. As a first example, FIGs. 16A and 16B show an example rendering of a heart 1600 with post-ischemic Ventricular Tachycardia (VT) characterized by endo-epicardial low or intermediate voltage area 1602 in which signal conduction is slowed down. This illustrates that measuring any prolonged potential inside or around the dense scar area may help identify potential isthmuses sustaining VT. The post-ischemic VT shown in FIG. 16A is characterized by an endo-epicardial low or intermediate voltage area in which signal conduction is slowed down. This illustrates that measuring any prolonged potential inside or around the dense scar area may help identify potential isthmuses sustaining VT. FIG. 16A illustrates the bipolar signal amplitude (Bi) variance in the various sectors of the heart 1600. FIG. 16A shows Bi ranges from 0.5 mV to 1.5 mV. FIG. 16B illustrates the Shortex Complex Interval (SCI) variance in the various sectors of the heart. As an example, SCI ranges from 15.0 msec to 171.00 msec with the SCI range of interest between 80 msec and 170 msec.

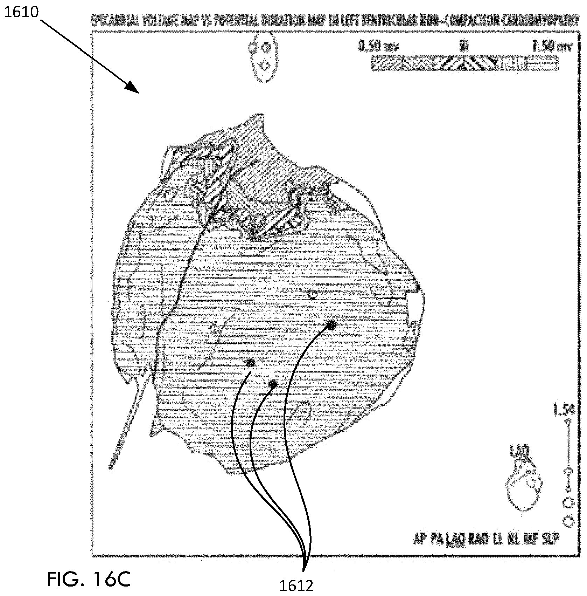

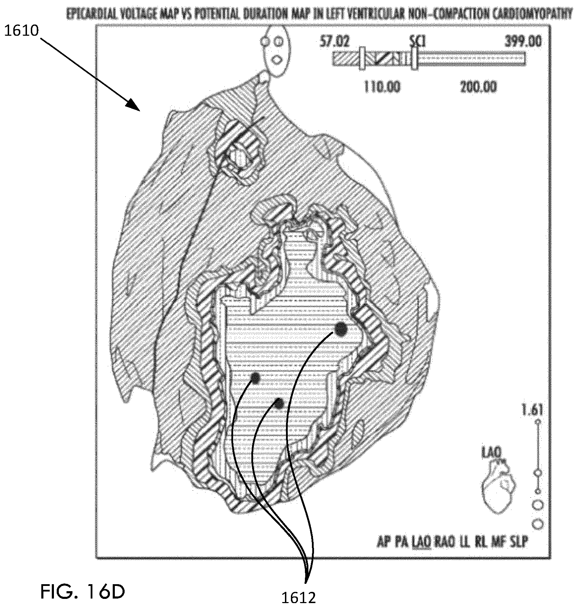

FIGs. 16C and 16D show an example rendering of a heart 1610 experiencing left ventricular non-compaction cardiomyopathy. More specifically, FIG. 16C shows an epicardial voltage map and FIG. 16D shows potential duration map (PDM). The three black circles in 1612 in FIGs. 16C and 16D are marked as abnormally prolonged potentials, e.g., potentials above 200 msec.

Abnormal tissue is generally characterized by low-voltage EGMs. However, initial clinical experience in endo-epicardial mapping indicates that areas of low-voltage are not always present as the sole arrhythmogenic mechanism in such patients. In fact, areas of low or medium-voltage may exhibit EGM fragmentation and prolonged activities during sinus rhythm, which corresponds to the critical isthmus identified during sustained and organized ventricular arrhythmias, e.g., applies only to non-tolerated ventricular tachycardias. Moreover, in many cases, EGM fragmentation and prolonged activities are observed in the regions showing a normal or near-normal voltage amplitude (>1-1.5 mV). Although the latter areas may be evaluated according to the voltage amplitude, they cannot be considered as normal according to the intracardiac signal, thus representing a true arrhythmogenic substrate. The 3D mapping may be able to localize the arrhythmogenic substrate on the endocardial and/or epicardial layer of the right/left ventricle, which may vary in distribution according to the extension of the main disease.

The substrate linked to these cardiac conditions is related to the presence of fragmented and prolonged EGMs in the endocardial and/or epicardial layers of the ventricular chambers (right and left). The 3D mapping system, such as CARTO®3, is able to localize the potential arrhythmogenic substrate of the cardiomyopathy in terms of abnormal EGM detection.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral artery, and then guided into the chamber of the heart of concern. A typical ablation procedure involves the insertion of a catheter having at least one electrode at its distal end, into a heart chamber. A reference electrode is provided, generally taped to the skin of the patient or by means of a second catheter that is positioned in or near the heart. RF (radio frequency) current is applied to the tip electrode of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the electrode also occurs as a result of conduction from the heated tissue to the electrode itself. If the electrode temperature becomes sufficiently high, possibly above 60 degrees C., a thin transparent coating of dehydrated blood protein can form on the surface of the electrode. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs and the catheter must be removed from the body and the tip electrode cleaned.

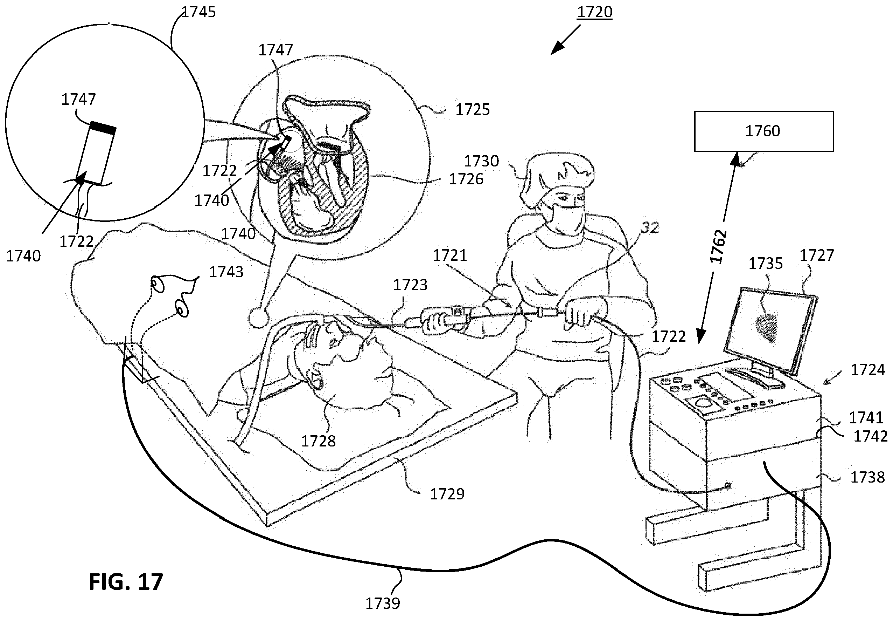

FIG. 17 is a diagram of an exemplary system 1720 in which one or more features of the disclosure subject matter can be implemented. All or parts of system 1720 may be used to collect information for a training dataset and/or all or parts of system 1720 may be used to implement a trained model. System 1720 may include components, such as a catheter 1740, that are configured to damage tissue areas of an intra-body organ. The catheter 1740 may also be further configured to obtain biometric data. Although catheter 1740 is shown to be a point catheter, it will be understood that a catheter of any shape that includes one or more elements (e.g., electrodes) may be used to implement the embodiments disclosed herein. System 1720 includes a probe 1721, having shafts that may be navigated by a physician 1730 into a body part, such as heart 1726, of a patient 1728 lying on a table 1729. According to embodiments, multiple probes may be provided, however, for purposes of conciseness, a single probe 1721 is described herein but it will be understood that probe 1721 may represent multiple probes. As shown in FIG. 17, physician 1730 may insert shaft 1722 through a sheath 1723, while manipulating the distal end of the shafts 1722 using a manipulator 1732 near the proximal end of the catheter 1740 and/or deflection from the sheath 1723. As shown in an inset 1725, catheter 1740 may be fitted at the distal end of shafts 1722. Catheter 1740 may be inserted through sheath 1723 in a collapsed state and may be then expanded within heart 1726. Cather 1740 may include at least one ablation electrode 1747 and a catheter needle 1748, as further disclosed herein.

According to exemplary embodiments, catheter 1740 may be configured to ablate tissue areas of a cardiac chamber of heart 1726. Inset 1745 shows catheter 1740 in an enlarged view, inside a cardiac chamber of heart 1726. As shown, catheter 1740 may include at least one ablation electrode 1747 coupled onto the body of the catheter. According to other exemplary embodiments, multiple elements may be connected via splines that form the shape of the catheter 1740. One or more other elements (not shown) may be provided and may be any elements configured to ablate or to obtain biometric data and may be electrodes, transducers, or one or more other elements.

According to embodiments disclosed herein, the ablation electrodes, such as electrode 1747, may be configured to provide energy to tissue areas of an intra-body organ such as heart 1726. The energy may be thermal energy and may cause damage to the tissue area starting from the surface of the tissue area and extending into the thickness of the tissue area.

According to exemplary embodiments disclosed herein, biometric data may include one or more of LATs, electrical activity, topology, bipolar mapping, dominant frequency, impedance, or the like. The local activation time may be a point in time of a threshold activity corresponding to a local activation, calculated based on a normalized initial starting point. Electrical activity may be any applicable electrical signals that may be measured based on one or more thresholds and may be sensed and/or augmented based on signal to noise ratios and/or other filters. A topology may correspond to the physical structure of a body part or a portion of a body part and may correspond to changes in the physical structure relative to different parts of the body part or relative to different body parts. A dominant frequency may be a frequency or a range of frequency that is prevalent at a portion of a body part and may be different in different portions of the same body part. For example, the dominant frequency of a pulmonary vein of a heart may be different than the dominant frequency of the right atrium of the same heart. Impedance may be the resistance measurement at a given area of a body part.

As shown in FIG. 17, the probe 1721, and catheter 1740 may be connected to a console 1724. Console 1724 may include a processor 1741, such as a general-purpose computer, with suitable front end and interface circuits 1738 for transmitting and receiving signals to and from catheter, as well as for controlling the other components of system 1720. In some embodiments, processor 1741 may be further configured to receive biometric data, such as electrical activity, and determine if a given tissue area conducts electricity. According to an embodiment, the processor may be external to the console 1724 and may be located, for example, in the catheter, in an external device, in a mobile device, in a cloud-based device, or may be a standalone processor.

As noted above, processor 1741 may include a general-purpose computer, which may be programmed in software to carry out the functions described herein. The software may be downloaded to the general-purpose computer in electronic form, over a network, for example, or it may, alternatively or additionally, be provided and/or stored on non-transitory tangible media, such as magnetic, optical, or electronic memory. The example configuration shown in FIG. 17 may be modified to implement the embodiments disclosed herein. The disclosed embodiments may similarly be applied using other system components and settings. Additionally, system 1620 may include additional components, such as elements for sensing electrical activity, wired or wireless connectors, processing and display devices, or the like.

According to an embodiment, a display connected to a processor (e.g., processor 1741) may be located at a remote location such as a separate hospital or in separate healthcare provider networks. Additionally, the system 1720 may be part of a surgical system that is configured to obtain anatomical and electrical measurements of a patient's organ, such as a heart, and performing a cardiac ablation procedure. An example of such a surgical system is the Carto®

system sold by Biosense Webster.

The system 1720 may also, and optionally, obtain biometric data such as anatomical measurements of the patient's heart using ultrasound, computed tomography (CT), magnetic resonance imaging (MRI) or other medical imaging techniques known in the art. The system 1720 may obtain electrical measurements using catheters, electrocardiograms (EKGs) or other sensors that measure electrical properties of the heart. The biometric data including anatomical and electrical measurements may then be stored in a memory 1742 of the mapping system 1720, as shown in FIG. 17. The biometric data may be transmitted to the processor 1741 from the memory 1742. Alternatively, or in addition, the biometric data may be transmitted to a server 1760, which may be local or remote, using a network 1662.

Network 1762 may be any network or system generally known in the art such as an intranet, a local area network (LAN), a wide area network (WAN), a metropolitan area network (MAN), a direct connection or series of connections, a cellular telephone network, or any other network or medium capable of facilitating communication between the mapping system 1720 and the server 1760. The network 1662 may be wired, wireless or a combination thereof. Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 1762.

In some instances, the server 1762 may be implemented as a physical server. In other instances, server 1762 may be implemented as a virtual server a public cloud computing provider (e.g., Amazon Web Services (AWS) ®).

Control console 1724 may be connected, by a cable 1739, to body surface electrodes 1743, which may include adhesive skin patches that are affixed to the patient 1730. The processor, in conjunction with a current tracking module, may determine position coordinates of the catheter 1740 inside the body part (e.g., heart 1726) of a patient. The position coordinates may be based on impedances or electromagnetic fields measured between the body surface electrodes 1743 and the electrode 1748 or other electromagnetic components of the catheter 1740. Additionally, or alternatively, location pads may be located on the surface of bed 1729 and may be separate from the bed 1729.

Processor 1741 may include real-time noise reduction circuitry typically configured as a field programmable gate array (FPGA), followed by an analog-to-digital (A/D) ECG (electrocardiograph) or EMG (electromyogram) signal conversion integrated circuit. The processor 1741 may pass the signal from an A/D ECG or EMG circuit to another processor and/or can be programmed to perform one or more functions disclosed herein.

Control console 1724 may also include an input/output (I/O) communications interface that enables the control console to transfer signals from, and/or transfer signals to electrode 1747.

During a procedure, processor 1741 may facilitate the presentation of a body part rendering 1735 to physician 1730 on a display 1727, and store data representing the body part rendering 1735 in a memory 1742. Memory 1742 may comprise any suitable volatile and/or non-volatile memory, such as random-access memory or a hard disk drive. In some embodiments, medical professional 1730 may be able to manipulate a body part rendering 1735 using one or more input devices such as a touch pad, a mouse, a keyboard, a gesture recognition apparatus, or the like. For example, an input device may be used to change the position of catheter 1740 such that rendering 1735 is updated. In alternative embodiments, display 1727 may include a touchscreen that can be configured to accept inputs from medical professional 1730, in addition to presenting a body part rendering 1735.

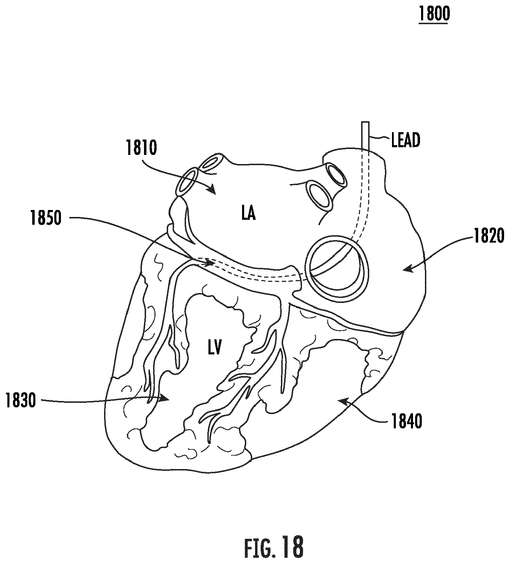

FIG. 18 illustrates a heart 1800. The heart 1800 is made up of four chambers - two upper chambers (atria) 1810, 1820 and two lower chambers (ventricles) 1830, 1840. The coronary sinus (CS) 1850 is a collection of veins joined together to form a large vessel that collects blood from the heart muscle and delivers less-oxygenated blood to the right atrium 1820.

The rhythm of the heart 1800 is normally controlled by the sinus node (not shown) located in the right atrium. The sinus node produces electrical impulses that normally start each heartbeat and acts as a natural pacemaker. From the sinus node, the electrical impulses travel across the atria 1810, 1820, causing the atria muscles to contract and pump blood into the ventricles 1830, 1840. The electrical impulses then arrive at a cluster of cells called the atrioventricular node (AV node) (not shown). The AV node is usually the only pathway for signals to travel from the atria 1810, 1820 to the ventricles 1830, 1840.

The AV node slows down the electrical signal before sending it to the ventricles 1830, 1840. The delay, even though slight, allows the ventricles 1830, 1840 to fill with blood. When electrical impulses reach the muscles of the ventricles, the muscles contract, causing the muscles to pump blood either to the lungs or to the rest of the body. In a healthy heart, this process usually goes smoothly, resulting in a normal resting heart rate of 60 to 100 beats a minute.

In a heart that has one of the disease states identified above, faulty electrical connections in the heart or abnormal areas of electrical activity trigger and sustain an abnormal rhythm. When this happens, the heart rate accelerates too quickly and doesn't allow enough time for the heart to fill before it contracts again. These ineffective contractions of the heart may cause light-headedness or dizziness because the brain may not be receiving enough blood and oxygen.

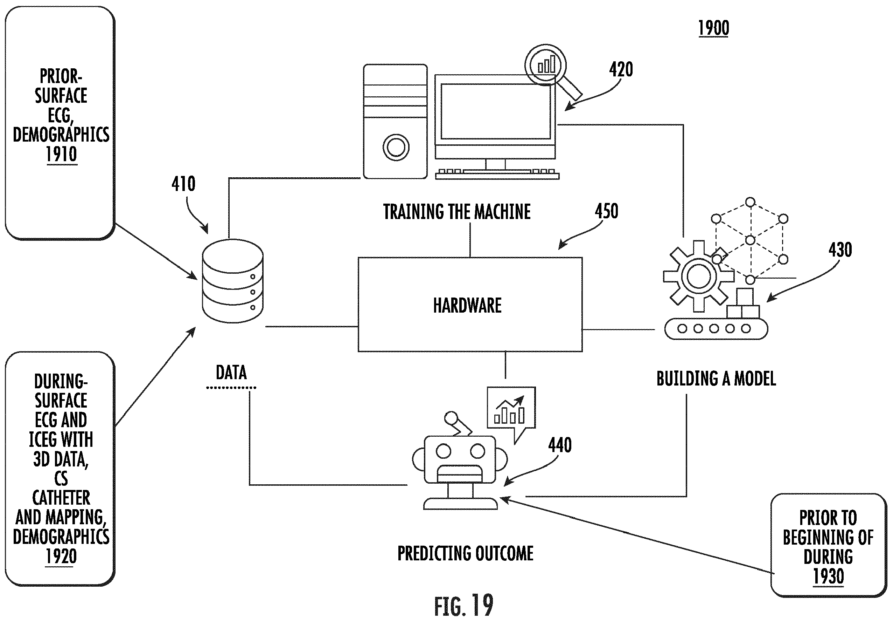

FIG. 19 illustrates a graphical depiction 1900 of the artificial intelligence system of FIG. 4 providing further detail related to predicting the steps of a cardiac ablation procedure. Depiction 1900 includes additional detail from FIG. 4. For example, depiction 1900 includes prior to procedure data 1910 that may be used to predict the steps required in the ablation. These prior to procedure data 1910 may include ECG and demographical information, for example. During procedure data 1920 is also included within depiction 1900. During procedure data 1920 may include surface ECG and ICEG information, may include 3D information, CS catheter and mapping information, and demographics. Depiction 1900 may include predicting outcomes 440 from FIG. 4, including predicting outcomes 1930 prior to a procedure, at the beginning of a procedure and during the procedure.

An ablation procedure of atrial fibrillation may include several steps. The first step is usually pulmonary vein isolation (PVI). In many cases PVI results a sinus rhythm that will last for several years. However, in some of the cases, additional ablation to PVI may be required in order to achieve a sinus rhythm. The ability to identify in advance of a procedure, or even during a procedure, the steps needed during the ablation procedure may allow the physician to plan and schedule in an efficient way. In addition, the additional knowledge concerning the procedure may lead to an improved ablation efficacy.

However, the prediction of the steps needed and the length of time needed for an ablation procedure, the time and complexity of the procedure is difficult. This causes issues and inefficiency in planning the schedule for the clinic or hospital where the ablation is performed. In addition, on occasion an ablation of PVI may result in a sinus rhythm, especially if cardioversion is used to obtain the sinus rhythm, where the arrhythmia recurs approximately 2-3 months following the ablation and a second procedure is needed. Being able to predict that PVI alone may not be effective is important and allows the physician to treat the patient more effectively.

In the present system, a prediction 1930 of the required steps in the ablation may be made prior to the procedure of the ablation steps that may be needed, and/or a prediction 1930 at the beginning of the procedure of the required steps in the ablation, such as immediately following the first mapping, of the ablation steps that may be needed. The present system and method may predict 1930 the steps needed at different time points related to the procedure, such prior to the ablation and during the ablation, for example.

A prediction model based on machine learning may be used as described herein above. The data 410 used for prediction may include, prior to the procedure 1910, such as surface ECG and demographics information, and during the procedure 1920, such as surface ECG and ICEG as well as 3D data, CS catheter / general mapping, and demographic information. Ground truth may include databases collected in large medical centers including the outcome of the procedure, at the end of the procedure and after a follow up of 12 months.

The present system and method may allow a more efficient plan to the procedure, and better scheduling of the physician's time and the laboratory time. A comparison to the baseline ECG may be performed in order to determine the case length based on the ECG. The efficiency of scheduling, the approach to the treatment plan and optimizing the approach may be achieved through the present system and method.

Premature ventricular contractions (PVCs) are extra heartbeats that begin in one of the heart's two lower pumping chambers (ventricles). Premature atrial contractions (PACs), also known atrial premature beats (APB), are a common cardiac arrhythmia characterized by premature heartbeats originating in the atria. While the sinoatrial node typically regulates the heartbeat during normal sinus rhythm, PACs & PVCs occur when another region of the ventricle/atria depolarizes before the sinoatrial node and thus triggers a premature heartbeat. These extra beats disrupt regular heart rhythm, sometimes causing a fluttering or a skipped beat in the chest. Because these beats are singular in nature it is not trivial to understand their origin during an electrocardiography intervention and often physicians need to stimulate the heart at numerous points in order to trigger the PVC's/PAC's. Consequently, the procedures are long, and not always conclusive. The existence of the PVC/PAC is commonly verified through a body surface ECG halter. However common methods are not sufficient to quickly and accurately localize the origins of the PVC/PAC. The present system and method use ML tools in combination with a methodological placement of the BS ECG leads in order to focus the physician towards the origin of the PVC/PAC. The present system and method may be utilized in performing atrial fibrillation, atrial flutter, general electrophysiology, SVT and simple arrhythmias, ventricular fibrillation, and ventricular tachycardia, for example.

A method, apparatus and system for patient-specific planning and guidance of an ablation procedure for cardiac arrhythmia (PVC/PAC) is disclosed. The ML algorithm collects data such as BS ECG, cardiac dimensions, patient demographics, patient dimensions, and previous mapping and ablation procedures, and, especially for premature ventricular contraction patients, the neural network may indicate where the PVC/PAC is originating, and is provided in a 3D view, for example. The present algorithm is implemented by learning from a database with existing PVC/PAC procedures, such as those with known origins.

In some embodiments, the BS ECG is collected using a specific apparatus (e.g. belt) that distribute the ECG leads in the same locations on the patient's body (pre-procedure and intra-procedure ECG). A patient-specific 3D heart model is generated based on a neural network analysis of pre-operative data. The pre-operative data may include body surface, such as 12 lead, ECG, cardiac chamber dimensions, patient demographics and previous mapping and/or ablation procedures.

The patient-specific 3D heart model is registered to a coordinate system of intra-operative mapping system used during the ablation procedure. One or more ablation site guidance maps are generated based on the registered patient-specific 3D heart model and intra-operative patient specific measurements acquired during the ablation procedure. The ablation site guidance maps are generated using a computational model of cardiac electrophysiology which is personalized by fitting parameters of the cardiac electrophysiology model using the intra-operative patient-specific measurements. The ablation site guidance maps are displayed by a display device (e.g., 3D mapping system) during the ablation procedure.

Prior to the procedure, pre-procedure localization of the origin of the PVC/PAC may be performed to save time during the procedure. Localizing the origin of the arrhythmia, such as a focal arrhythmia or focal atria, and a micro-macro reentry-based arrhythmia, including the premature beat and delay for next subsequent beat. The QRS (positive / negative) may provide input for deducing where the arrhythmia source is located. The direction of the QRS PT for the sinus rhythm may provide additional information related to the origin. The ICEG, demographics and medical dimensions of the heart, as well as catheter positions, respiration data, anatomical position based on heart chambers (CT/MRI/FAM 3D heart model, and the like, may provide additional detail on the origin. Once the location is identified, a separate mapping for each arrhythmia source may be generated. Presentation of this origin on a 3D, patient specific, heart model may be provided in the present system and method. This 3D model is registered to the coordinate system of the 3D mapping system used for the procedure. This present system and method may allow a physician to skip or perform a partial mapping based on the knowledge of the location of the arrhythmia source.

In addition, the present invention is designed to collect data prior to EP procedure. This data may include magnetic resonance (MR), computed tomography (CT), positron emission tomography (PET), ultrasound information, and heart wall thickness based on segmentation of previous maps, body surface (BS) ECG (12 leads), and pseudo BS ECG(Vest). The data from these studies may be used as an input for an artificial intelligence-based classifier, as described in Figure 4, for non-sustained tachycardia source for epicardial and/or endocardial. The training of the collected data and the source may be provided by a physician and may include past studies and tests. The benefit of arrhythmia source prior knowledge is important in the preparation and setup for epicardial EP procedure and is different from endocardial procedure.

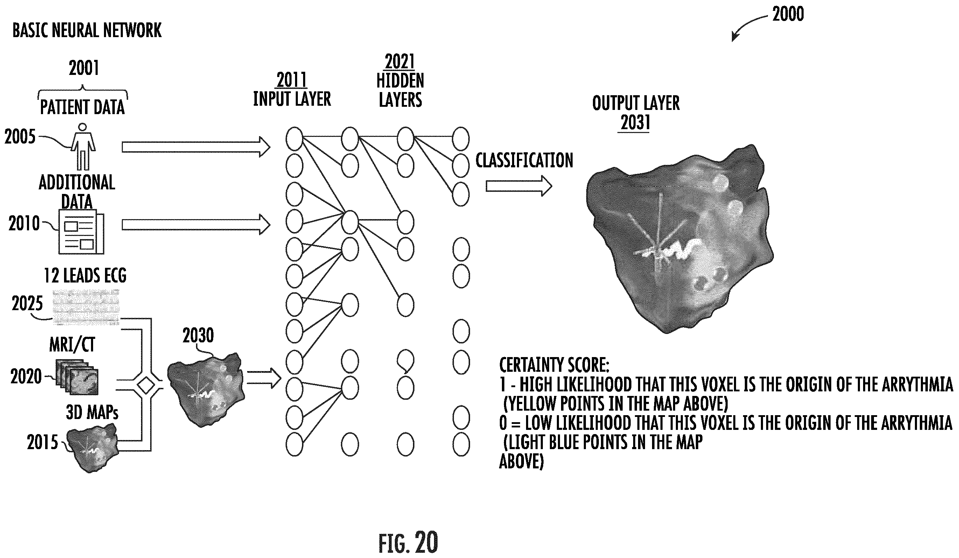

A system and method for determining the source or sources of a cardiac arrhythmia may be based on a myriad of inputs 2001. As illustrated in FIG. 20 described below, inputs 2001 may include a 12 lead ECG 2025 or similar body surface ECG 2025 data representing the medical condition (arrhythmia) and a 3D representation of the heart (from MRI/CT 2020 or a 3D mapping system 2030. Inputs 2001 may also include patient data 2005 that may include demographic data, electro-anatomical information from a 3D mapping system 2030, CT scan and/or MRI scans (collectively 2020) and Intracardiac and Body Surface related information (full list disclosed below). The output 2031 of the system and method may include an output indicating the origin or origins of the arrythmia on the 3D model, each origin includes a certainty score.

FIG. 20 illustrates a system 2000 using a neural network in accordance with the embodiments described herein. System 2000 includes patient data 2005, additional data 2010, and 3D mappings 2015, MRI/CT 2020, ECG 2025 including 12-lead ECGs, for example, as inputs 2001. 3D Mappings 2015 may be combined with MRI/CT 2020 and ECG 2025 to form a composite mapping 2030.

This input 2001 may include 3D mappings and imaging from several algorithms that analyze readings from the patient. For example, the input 2001 may include 12-Lead Body Surface ECG from the patient manifesting the arrhythmia and a 3D representation of the heart from either CT, MRI or a 3D mapping system. This data may include data per electrode/channel provided in slices (e.g. CT slices) or as a 3D shell composed, for examples from triangles (e.g. 3D mesh object). The 12-Leads ECG with the arrythmia can be provided digitally or as a picture of the ECG.

The patient data 2005 may include demographic information, age, gender, weight, height, body mass index, ethnicity and other patient specific details including cardiac chamber main axis lengths (width, height, length), left ventricular ejection fraction, hypertension and diabetes mellitus. Other baseline comorbidities may be included in the additional data 2010 including sleep apnea, coronary artery disease, valvular heart disease (e.g. mitral regurgitation), congestive heart disease. The patient medical history may be included in the patient data 2005, and may include arrhythmia history, symptoms, and documented method, time since first diagnosis, anti-arrhythmia drug (AAD) history, previous cardiac ablations, anticoagulation, CHA2DS2-VASc Score, history of Thrombotic Diseases, New York Heart Association (NYHA) Grade of Cardiac Function, history of Hemorrhagic Diseases, HAS-BLED, respiration pattern, ventricular cycle length, atrial cycle length.

Data per electrode/channel and analyses performed on this data (e.g. derivatives, algorithmic calculations) may include local activation time, impedance over a period of time, impedance changes over time, rate of location changes (derivative of the position over time), maximal peak to peak voltage, unipolar and bipolar measurements from far away regions (not the point or immediate surrounding), and the start and end time points of the time period in which the catheter was located at that point. Such time tagged data may provide insight because different points in the 3D mapping were calculated based on measurements done at different times. If a certain point value was triangulated by the algorithm rather than obtained from a visit of the catheter at that point, then a triangulation of relevant time points may be used. Data per chamber (can be epicardial map and / or endocardial map) may include wall motion from ULS, doppler from ULS, scar zones from MRI, chamber dimensions, Cycle Length Map, persistent Atrial Fibrillation Focal Sources Map (e.g. CARTOFINDER, or equivalent), persistent Atrial Fibrillation Rotational sources map (e.g. CARTOFINDER, or equivalent), Cycle Length maps, reentrant / Fibrillation activation mapping (e.g. Coherent, or equivalent), ripple map, CFAE, ECG Fractionation, and 3D model of cardiac tissue, where each voxel has an indication of tissue elasticity at that voxel, based on data from Ultrasound readings.

The output 2031 is a set of voxels representing cardiac tissue, where each voxel has a certainty score. The certainty score may include a number from 0 to 1 with 1 = highest likelihood that the voxel is the origin of the arrythmia, <1 & >0 intermediate scoring of likelihood of origin and 0 = low likelihood that the voxel is the origin of the arrythmia. The certainty score may be provided between 0 and 1, between 0% to 100% or in other scoring methods.

Between the input 2001 and the output 2031, the 3D mappings may be combined as set forth to achieve a composite mapping 2030. The composite mapping 2030 may be input to the neural network 2000 along with patient data 2005 and any additional data 2010 as described above. The neural network 2000 may include an input layer 2011, hidden layers 2021, such as layers described herein, and output layer 2031.

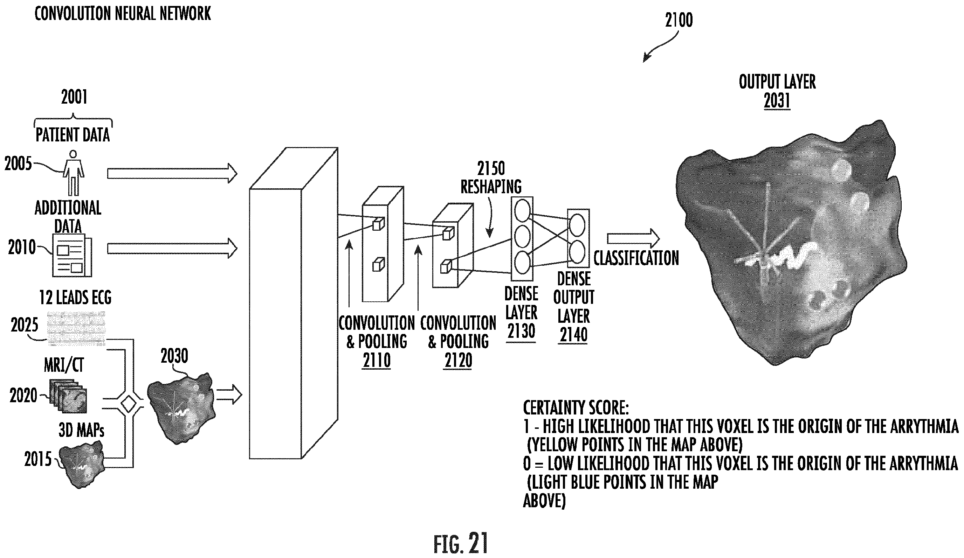

Similar to the neural network of FIG. 20, the system 2100 of FIG. 21 utilizes a neural network in accordance with the embodiments described herein. System 2100 includes patient data 2005, additional data 2010, and 3D mappings 2015, including, for example, MRI/CT 2020, ULS and other 3D mappings based on catheter 715 and ECGs such as 12-lead ECGs 2025. This input data 2001 is that which is input into the other embodiments including system 2000.

Between the input data 2001 and the output 2031, the 3D mappings may be combined as set forth to achieve a composite mapping 2030. The composite mapping 2030 may be input to the neural network 2100 along with patient data 2005 and any additional data 2010 as described above. The neural network 2100 may include a first convolution and pooling 2110, followed by a second convolution and pooling 2120. The output from the convolutions and pooling 2110, 2120 may reshaped using reshaping 2150 and the reshaped data input to a dense layer 2130 and a dense output layer 2140 in series. Once the data is classified, the data reaches an output layer similar to output layer 2031. The output layer provides the output.

The output 2031 is a set of voxels representing cardiac tissue, where each voxel has a certainty score. The certainty score may include a number from 0 to 1 with 1 = highest likelihood that the voxel is the origin of the arrythmia, <1 & >0 intermediate scoring of likelihood of origin and 0 = low likelihood that the voxel is the origin of the arrythmia. The certainty score may be provided between 0 and 1.

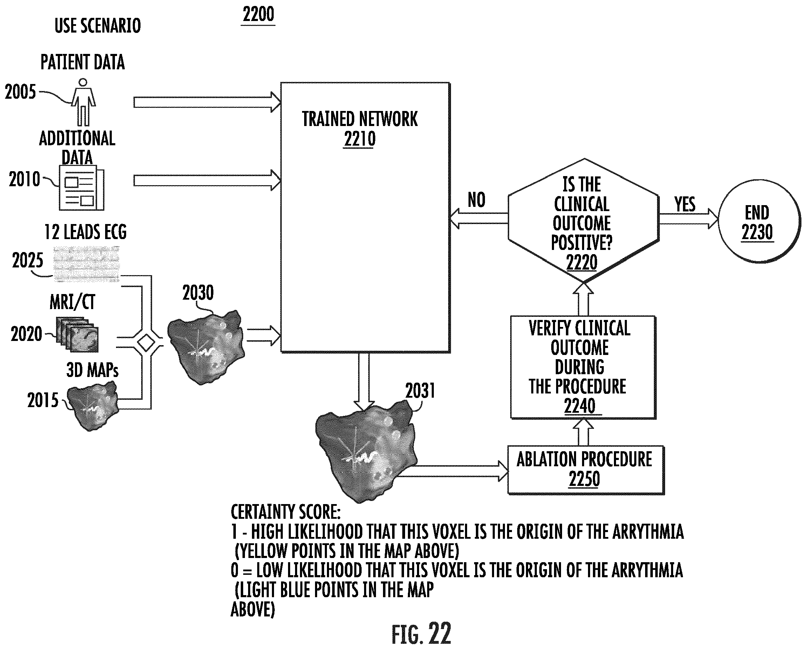

FIG. 22 illustrates an illustration of the use of the systems described herein. As illustrated system 2200 includes inputs described herein. These include patient data 2005, additional data 2010, ECG 2025, MRI/CT 202 and 3d mappings 2015. As described above, ECG 2025, MRI/CT 2020 and 3d mappings 2015 may be combined to produce a composite mapping 2030.

The composite mapping 2030 along with the patient data 2005 and additional data 2010 may be fed into a trained network 2210, such as the neural networks of FIGs. 20 and 21. As described with respect to those figures, the output 2031 is an image of a heart with certainty scores associated with each voxel. Output 2031 is then fed as an input into the ablation procedure 2250. The output provides information on the origins of the arrythmia as described. The clinical outcome during the procedure is verified at step 2240. A determination on the outcome occurs at step 2220. If the outcome is determined at step 2220 to be positive, the procedure ends at step 2230. If the outcome is determined to be negative at step 2220, this information is fed back into the trained network 2210 to improve the learning of the system. In accordance with one or more embodiments, the technical effects and benefits of the system and method for identifying the origin of the arrythmia to provide input into the produce to treat the arrythmia.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random-access memory (RAM), a register, cache memory, semiconductor memory devices, magnetic media such as internal hard disks and removable disks, magneto-optical media, and optical media such as CD-ROM disks, and digital versatile disks (DVDs). A processor in association with software may be used to implement a radio frequency transceiver for use in a WTRU, UE, terminal, base station, RNC, or any host computer.

**Claims**

1. A method of aiding a physician in locating the origin of an arrythmia for patients with non-sustained tachycardia, comprising:

receiving data at a machine, from at least one device, the data including information relating to a desired location for performing an ablation;

generating, by the machine, an optimal location for performing the ablation based upon the data; and

providing an optimal location for performing the ablation.

2. A system of aiding a physician in locating the origin of an arrythmia for patients with non-sustained tachycardia, comprising:

an input/out device for receiving data, from at least one device, the data including information relating to a desired location for performing an ablation; and

a processor for generating an optimal location for performing the ablation based upon the received data;

the input/out device outputting the optimal location for performing the ablation.

3. The method of claim 1 or the system of claim 2 with certainty score for the origins of the arrythmia.

4. The method or system of claim 3 wherein the certainty score ranges from 0 to 1.

5. The method or system of claim 3 wherein the certainty score is assigned per voxel in the output associated with the optimal location.

6. The system of claim 1 wherein the processor is provided feedback associated with the outcome of the arrythmia procedure.

7. The system of claim 1 further receiving data during the ablation procedure, and the processor calculating new ablation origins.

8. The method of claim 3 wherein the machine is provided feedback associated with the outcome of the arrythmia procedure.

9. The method of claim 1 with an option to obtain new origins during the ablation procedure.

10. The method of claim 1 or the system of claim 2 wherein the data include patient data.

11. The method of claim 1 or the system of claim 2 wherein the data include additional data.

12. The method of claim 1 or the system of claim 2 wherein the data includes at least one of an ECG, MRI/CT, and 3D mapping.

13. The method or system of claim 12 wherein the at least one of an ECG, MRI/CT, and 3D mapping are combined to produce a composite map.

PATIENT

MONITORING AND PROCESSING APPARATUS

SENSOR

PROCESSOR

UI SENSOR

MEMORY

TRANSCEIVER

104

102

112

114

116

118

122

LOCAL COMPUTING DEVICE

106

NETWORK

110

NETWORK

120

REMOTE COMPUTING SYSTEM

108

100

FIG. 1

FIG. 2

EP 3 965 115 A1

300

FIG. 3

FIG. 4

500

```
┌─────────────────────────────────────┐
│      Collect Data From Hardware      │─── 510
└─────────────────────────────────────┘
                   │
                   ▼
┌─────────────────────────────────────┐
│     Train a Machine on the Hardware  │─── 520
└─────────────────────────────────────┘
                   │
                   ▼
┌─────────────────────────────────────┐
│             Build a Model            │─── 530
└─────────────────────────────────────┘
                   │
                   ▼
┌─────────────────────────────────────┐
│    Predict Outcomes of the Hardware  │─── 540
└─────────────────────────────────────┘
```

# FIG. 5

**610**

| Weather | Play |
|---|---|
| Sunny | No |
| Overcast | Yes |
| Rainy | Yes |
| Sunny | Yes |
| Sunny | Yes |
| Overcast | Yes |
| Rainy | No |
| Rainy | No |
| Sunny | Yes |
| Rainy | Yes |
| Sunny | No |
| Overcast | Yes |
| Overcast | Yes |
| Rainy | No |

**620**

| Frequency Table | | |
|---|---|---|
| Weather | No | Yes |
| Overcast | | 4 |
| Rainy | 3 | 2 |
| Sunny | 2 | 3 |
| Grand Total | 5 | 9 |

**630**

| Likelihood Table | | | | |
|---|---|---|---|---|
| Weather | No | Yes | | |
| Overcast | | 4 | =4/14 | 0.29 |
| Rainy | 3 | 2 | =5/14 | 0.36 |
| Sunny | 2 | 3 | =5/14 | 0.36 |
| All | 5 | 9 | | |
| | =5/14 | =9/14 | | |
| | 0.36 | 0.64 | | |

FIG. 6

FIG. 7

FIG. 8

EP 3 965 115 A1

FIG. 9

FIG. 10

FIG. 11

EP 3 965 115 A1

FIG. 12

**FIG. 13**

FIG. 14

FIG. 15

FIG. 16A

FIG. 16B

EPICARDIAL VOLTAGE MAP VS POTENTIAL DURATION MAP IN LEFT VENTRICULAR NON-COMPACTION CARDIOMYOPATHY

0.50 mv     BI     1.50 mv

1610

1612

1.54

LAO

AP PA LAO RAO LL RL MF SLP

FIG. 16C

FIG. 16D

FIG. 17

FIG. 18

PRIOR-
SURFACE
ECG,
DEMOGRAPHICS
1910

DURING-
SURFACE
ECG AND
ICEG WITH
3D DATA,
CS
CATHETER
AND MAPPING,
DEMOGRAPHICS
1920

410

DATA

1900

420

TRAINING THE MACHINE

450

HARDWARE

430

BUILDING A MODEL

440

PREDICTING OUTCOME

PRIOR TO
BEGINNING OF
DURING
1930

FIG. 19

FIG. 20

CONVOLUTION NEURAL NETWORK

2100

OUTPUT LAYER
2031

2001
PATIENT DATA

2005 — ○ (person figure)

ADDITIONAL DATA

2010 —

12 LEADS ECG

2025 —

MRI/CT

2020 —

3D MAPs

2015 —

2030

2150
RESHAPING

CONVOLUTION & POOLING 2110

CONVOLUTION & POOLING 2120

DENSE LAYER 2130

DENSE OUTPUT LAYER 2140

CLASSIFICATION

CERTAINTY SCORE:
1 - HIGH LIKELIHOOD THAT THIS VOXEL IS THE ORIGIN OF THE ARRYTHMIA
(YELLOW POINTS IN THE MAP ABOVE)
0 = LOW LIKELIHOOD THAT THIS VOXEL IS THE ORIGIN OF THE ARRYTHMIA
(LIGHT BLUE POINTS IN THE MAP
ABOVE)

FIG. 21

EP 3 965 115 A1

**USE SCENARIO**

2200

**PATIENT DATA**

2005

**ADDITIONAL DATA**

2010

**12 LEADS ECG**

2025

**MRI/CT**

2020

**3D MAPs**

2015

2030

**TRAINED NETWORK**
2210

2031

NO

**IS THE CLINICAL OUTCOME POSITIVE?**
2220

YES

**END**
2230

**VERIFY CLINICAL OUTCOME DURING THE PROCEDURE**
2240

**ABLATION PROCEDURE**
2250

CERTAINTY SCORE:
1 - HIGH LIKELIHOOD THAT THIS VOXEL IS THE ORIGIN OF THE ARRYTHMIA (YELLOW POINTS IN THE MAP ABOVE)
0 = LOW LIKELIHOOD THAT THIS VOXEL IS THE ORIGIN OF THE ARRYTHMIA (LIGHT BLUE POINTS IN THE MAP ABOVE)

FIG. 22

Europäisches
Patentamt

European
Patent Office

Office européen
des brevets

# EUROPEAN SEARCH REPORT

Application Number

**EP 21 19 4504**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/038251 A1 (PACHON MATEOS JOSE C [BR] ET AL) 15 February 2007 (2007-02-15) * paragraph [0053] – paragraph [0128] * ----- | 1-13 | INV. G16H20/40 |
| A | EP 2 460 486 A1 (BIOSENSE WEBSTER INC [US]) 6 June 2012 (2012-06-06) * paragraph [0023] – paragraph [0043] * ----- | 1-13 | |

**TECHNICAL FIELDS
SEARCHED      (IPC)**

G16H

The present search report has been drawn up for all claims

| Place of search | Date of completion of the search | Examiner |
|---|---|---|
| Munich | 21 January 2022 | Rinelli, Pietro |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

........................................................................

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 21 19 4504

This annex lists the patent family members relating to the patent documents cited in the above-mentioned European search report.
The members are as contained in the European Patent Office EDP file on
The European Patent Office is in no way liable for these particulars which are merely given for the purpose of information.

21-01-2022

| Patent document cited in search report | | Publication date | Patent family member(s) | | Publication date |
|---|---|---|---|---|---|
| US 2007038251 | A1 | 15-02-2007 | US | 2007038251 A1 | 15-02-2007 |
| | | | US | 2011060327 A1 | 10-03-2011 |
| | | | US | 2011098699 A1 | 28-04-2011 |
| | | | US | 2012184950 A1 | 19-07-2012 |
| | | | US | 2012191087 A1 | 26-07-2012 |
| | | | US | 2012191088 A1 | 26-07-2012 |
| EP 2460486 | A1 | 06-06-2012 | AU | 2011253826 A1 | 21-06-2012 |
| | | | CA | 2760928 A1 | 06-06-2012 |
| | | | CN | 102551878 A | 11-07-2012 |
| | | | DK | 2460486 T3 | 05-10-2015 |
| | | | EP | 2460486 A1 | 06-06-2012 |
| | | | IL | 216762 A | 31-07-2016 |
| | | | JP | 2012135613 A | 19-07-2012 |
| | | | RU | 2011149451 A | 10-06-2013 |
| | | | US | 2012143097 A1 | 07-06-2012 |
| | | | US | 2018310991 A1 | 01-11-2018 |

EPO FORM P0459

For more details about this annex : see Official Journal of the European Patent Office, No. 12/82

**REFERENCES CITED IN THE DESCRIPTION**

**Patent documents cited in the description**

- US 63073530